# EUROPEAN PATENT APPLICATION

(11) **EP 0 911 036 A2**
(43) Date of publication of application: **28.04.1999**
(21) Application number: 98122950.3
(22) Date of filing: 10.02.1993
(51) Int. Cl.: A61K 39/385, A61K 39/44, A61K 39/40, A61K 39/42, C08B 37/00, C08B 37/02, C07K 17/10, A61K 47/36, A61K 47/42

(54) **Dual carrier immunogenic construct**

(30) Priority: 11.02.1992 US 834067
(62) Divisional of application: 93906033.1
(71) Applicant: HENRY M. JACKSON FOUNDATION FOR THE ADVANCEMENT OF MILITARY MEDICINE, Rockville, MD 20852 (US)
(72) Inventor: Mond, James J., Potomac, MD 20854 (US); Lees, Andrew, Baltimore, MD 21218 (US)
(74) Representative: Grünecker, Kinkeldey, Stockmair & Schwanhäusser Anwaltssozietät

(57) **Abstract**

A dual carrier immunogenic construct comprised of at least one primary carrier comprising a large molecular weight molecule of greater than 70 kD molecular weight and at least one secondary carrier comprising a T-dependent antigen conjugated to a primary carrier. The dual carrier immunogenic construct may further comprise moieties such as haptens and antigens. Such immunogenic constructs are suitable for use in the diagnosis, treatment, and prevention of diseases.

## Description

### I. GOVERNMENT INTEREST

The invention described herein may be manufactured, licensed and used for governmental purposes without the payment of any royalties to us thereon.

### II. FIELD OF THE INVENTION

This invention relates to a dual carrier immunogenic construct that enhances the effectiveness of active immunization for animals and humans and for development of antibodies to be used for passive immunoprophylaxis or therapy and as scientific or diagnostic reagents.

### III. BACKGROUND OF THE INVENTION

In the process of vaccination, medical science uses the body's innate ability to protect itself against invading agents by immunizing the body with antigens that will not cause the disease but will stimulate the formation of antibodies that will protect against the disease. For example, dead organisms are injected to protect against bacterial diseases such as typhoid fever and whooping cough, toxins are injected to protect against tetanus and botulism, and attenuated organisms are injected to protect against viral diseases such as poliomyelitis and measles.

It is not always possible, however, to stimulate antibody formation merely by injecting the foreign agent. The vaccine preparation must be immunogenic, that is, it must be able to induce an immune response. Certain agents such as tetanus toxoid are innately immunogenic, and may be administered in vaccines without modification. Other important agents are not immunogenic, however, and must be converted into immunogenic molecules before they can induce an immune response.

The immune response is a complex series of reactions that can generally be described as follows:
1. the antigen enters the body and encounters antigen-presenting cells which process the antigen and retain fragments of the antigen on their surfaces;
2. the antigen fragment retained on the antigen presenting cells are recognized by T cells that provide help to B cells; and
3. the B cells are stimulated to proliferate and divide into antibody forming cells that secrete antibody against the antigen.

Most antigens only elicit antibodies with assistance from the T cells and, hence, are known as T-dependent (TD). These antigens, such as proteins, can be processed by antigen presenting cells and thus activate T cells in the process described above. Examples of such T-dependent antigens are tetanus and diphtheria toxoids.

Some antigens, such as polysaccharides, cannot be properly processed by antigen presenting cells and are not recognized by T cells. These antigens do not require T cell assistance to elicit antibody formation but can activate B cells directly and, hence, are known as T-independent antigens (TI). Such T-independent antigens include H. influenzae type b polyribosyl-ribitol-phosphate and pneumococcal capsular polysaccharides.

T-dependent antigens vary from T-independent antigens in a number of ways. Most notably, the antigens vary in their need for an adjuvant, a compound that will nonspecifically enhance the immune response. The vast majority of soluble T-dependent antigens elicit only low level antibody responses unless they are administered with an adjuvant. It is for this reason that the standard DPT vaccine (diptheria, pertussis, tetanus) is administered with the adjuvant alum. Insolubilization of TD antigens into an aggregated form can also enhance their immunogenicity, even in the absence of adjuvants. (Golub ES and WO Weigle, J. Immunol. 102:389, 1969) In contrast, T-independent antigens can stimulate antibody responses when administered in the absence of an adjuvant, but the response is generally of lower magnitude and shorter duration.

Four other differences between T-independent and T-dependent antigens are:
a) T-dependent antigens can prime an immune response so that a memory response can be elicited upon secondary challenge with the same antigen. Memory or secondary responses are stimulated very rapidly and attain significantly higher titers of antibody than are seen in primary responses. T-independent antigens are unable to prime the immune system for secondary responsiveness.
b) The affinity of the antibody for antigen increases with time after immunization with T-dependent but not T-independent antigens.
c) T-dependent antigens stimulate an immature or neonatal immune system more effectively than T-independent antigens.
d) T-dependent antigens usually stimulate IgM, IgG1, IgG2a, and IgE antibodies, while T-independent antigens stimulate IgM, IgG1, IgG2b, and IgG3 antibodies.

These characteristics of T-dependent vs. T-independent antigens provide both distinct advantages and disadvantages in their use as effective vaccines. T-dependent antigens can stimulate primary and secondary responses which are long-lived in both adult and in neonatal immune systems, but must frequently be administered with adjuvants. Thus, vaccines have been prepared using only an antigen, such as diphtheria or tetanus toxoid, but such vaccines may require the use of adjuvants, such as alum for stimulating optimal responses. Adjuvants are often associated with toxicity and have been shown to nonspecifically stimulate the immune system, thus inducing antibodies of specificities that may be undesirable.

Another disadvantage associated with T-dependent antigens is that very small proteins, such as peptides, are rarely immunogenic, even when administered with adjuvants. This is especially unfortunate because many synthetic peptides are available today that have been carefully synthesized to represent the primary antigenic determinants of various pathogens, and would otherwise make very specific and highly effective vaccines.

In contrast, T-independent antigens, such as polysaccharides, are able to stimulate immune responses in the absence of adjuvants. Unfortunately, however, such T-independent antigens cannot stimulate high level or prolonged antibody responses. An even greater disadvantage is their inability to stimulate an immature or B cell defective immune system (Mond J.J., Immunological Reviews 64:99, 1982) (Mosier DE, et al., J. Immunol. 119:1874, 1977). Thus, the immune response to both T-independent and T-dependent antigens is not satisfactory for many applications.

With respect to T-independent antigens, it is critical to provide protective immunity against such antigens to children, especially against polysaccharides such as H. influenzae and S. pneumoniae. With respect to T-dependent antigens, it is critical to develop vaccines based on synthetic peptides that represent the primary antigenic determinants of various pathogens.

One approach to enhance the immune response to T-independent antigens involves conjugating polysaccharides such H. influenzae PRP (Cruse J.M., Lewis R.E. Jr. ed., Conjugate vaccines in Contributions to Microbiology and Immunology, vol. 10, 1989) or oligosaccharide antigens (Anderson PW, et al., J. Immunol. 142:2464, 1989) to a single T-dependent antigen such as tetanus or diphtheria toxoid. Recruitment of T cell help in this way has been shown to provide enhanced immunity to many infants that have been immunized. Unfortunately, only low level antibody titers are elicited, and only some infants respond to initial immunizations. Thus, several immunizations are required and protective immunity is often delayed for months. Moreover, multiple visits to receive immunizations may also be difficult for families that live distant from medical facilities (especially in underdeveloped countries). Finally, babies less than 2 months of age may mount little or no antibody response even after repeated immunization.

The current solution for protein or peptide T-dependent antigens is similarly disadvantageous. T-dependent antigens are often incorporated into adjuvants or other delivery systems. Such an approach, however, may be toxic or may induce non-specific enhancement of the antibody response (Dancey GF, et al., J. Immunol. 122:638, 1979).

Moreover, these approaches with both T-dependent and T-independent antigens incorporate only a single T-dependent carrier to potentiate the immune response. Such approaches do not maximize recruitment of T-cell help. Moreover, these methods are extraordinarily limited and confined by the inability to administer multiple antigens on one carrier, and thus require numerous injections.

In another approach, investigators have conjugated a hapten such as Trinitrophenyl (TNP) to a T-independent carrier such as Ficoll® (an inert synthetic non-ionized high molecular weight polymer) of molecular weight 400K. Such a conjugate has been found to stimulate a T-independent response in mice in the absence of adjuvant (Mosier DE, et al., J. Exp. Med. 139:1354 (1974)). This conjugate alone, however, could not stimulate immune responses in neonatal mice or in B cell immune defective mice (Mosier DE, et al., J. Immunol. 119:1874, 1977). Responses of immune defective mice to this conjugate could only be induced in the presence of a particular adjuvant (Ahmad A and Mond JJ, J. Immunol. 136:1223, 1986). This is disadvantageous for the reasons discussed previously.

In a further study, TNP was conjugated onto insoluble particles and found to be an effective in vitro immunogen for neonatal mice and immune defective mice, but only at very high density of hapten per bead (Mond JJ, et al., J. Immunol. 123:239, 1979). Another laboratory, Dintzis et al., demonstrated that the ratio of hapten to carrier, as well as the molecular mass of the carrier, strongly influences immunogenicity of a T independent conjugate and the antibody responses it stimulates (Dintzis RZ, et al., J. Immunol. 143:1239, 1989).

Another attempted conjugate involved an anti-immunoglobulin antibody (anti-Ig) conjugated to a dextran (a glucose polymer) of high molecular weight (2x10⁶ daltons) to form an "anti-Ig Dex" conjugate. The conjugate was found to activate neonatal and mature B cells as well as B cells from immune defective mice at very low concentrations (Brunswick M, et al., J. Immunol. 140:3364, 1988). However, since anti-Ig Dex stimulates little or no T cell derived help and since it activates all B cells without regard to specificity, it could not provide an effective vehicle for a vaccine.

None of these approaches solved the problem because the constructs optimized only one type of immune response. For example, low molecular weight haptens conjugated onto T-independent carriers will optimize only the T-independent component of the anti-hapten response, and poorly immunogenic T-independent molecules conjugated onto T dependent carriers will have to rely on stimulation of T-dependent immunity and in soluble form this complex may be very limiting in activating T cells. Thus, there remains a need in the art for constructs that optimize both components. Such a construct would optimize both the T-independent component to specifically stimulate high levels of activation in antigen specific B cells as well as optimize the T-dependent component to simultaneously recruit T cell help. In addition, such a dual construct would rapidly induce high levels of antibody to both T dependent and T independent antigens in neonates, adults and immunodeficient animals and humans.

There is also a need in the art for a construct to which multiple antigens could be attached so that a number of antigens could be presented in one injection. A vaccine that could immunize an individual with multiple antigens on a single construct would be extremely valuable.

In sum, there is a need in the art (a) for a construct that optimizes both T-independent and T-dependent antigen components to stimulate a very rapid and large antibody response that will persist over long periods of time in both children and adults, as well as in individuals that have immunodeficiencies (b) for a construct to which multiple antigens or other immune enhancing adjuvants could be attached; and (c) for a construct that would rapidly stimulate antibodies, either monoclonal or polyclonal, which could be employed for passive immunoprophylaxis or therapy, and for diagnostic, or research purposes.

### IV. SUMMARY OF THE INVENTION

The present invention overcomes the problems and disadvantages of the prior constructs by providing a dual carrier immunogenic construct that improves immunogenicity. The dual carrier immunogenic construct comprises at least one primary carrier that is a large molecular weight molecule conjugated to at least one secondary carrier that is a T-dependent antigen. Such a primary carrier enables presentation of multiple copies of the secondary carrier at a relatively high antigenic density to both T and B cells. In addition, such a large backbone matrix acts as an efficient carrier for many and different secondary carriers so that a single construct could contain multiple antigenic specificities. In a preferred embodiment, the primary carrier is a large molecular weight T-independent antigen that can itself directly and potently activate B cells and that can serve as a large but relatively nondegradable backbone to carry many secondary carriers.

The secondary carrier is a T-dependent antigen. As a T-dependent antigen, the secondary carrier activates and recruits T cells to augment antibody production to itself as well as to other determinants which may be conjugated to itself or to the primary carrier. Multiple copies of secondary carriers may be conjugated to the primary carrier to significantly enhance T-cell activation and thereby augment antibody production against the secondary carrier.

In a preferred embodiment, the present invention also includes at least one moiety, such as a hapten and antigen, conjugated to a primary carrier and/or a secondary carrier. Conjugation permits the moiety to benefit from the T-cell help generated by the secondary carrier.

The dual carrier immunogenic construct of the invention also permits the conversion of non-immunogenic or poorly immunogenic molecules into strongly immunogenic molecules by conjugating the molecules onto the dual carrier construct. In addition, it is possible to conjugate immune enhancing adjuvants onto any of the carriers to further enhance immunogenicity. Preferably, such a dual carrier immunogenic construct is prepared from non-toxic components. In addition, such dual carrier immunogenic construct may reduce alterations of the antigenic sites, since protein conjugation to the primary carrier, such as dextran, involves minimal alteration to the carrier.

Finally, the dual carrier immunogenic construct of the invention can be applied to therapy, prophylaxis, diagnosis, and research.

Additional advantages and aspects of the invention will be set forth in the detailed description which follows, derived from that detailed description, or learned by practice of the invention.

To achieve the advantages and in accordance with the purpose of the invention, as embodied and broadly described in this specification, the invention comprises a dual carrier immunogenic construct made up of at least one primary carrier that is a large molecular weight molecule of greater than 70 KD molecular weight conjugated to at least one secondary carrier that is a T-dependent antigen. The immunogenicity of the construct is greater than at least one carrier alone. In a preferred embodiment, the primary carrier is a T-independent antigen and the secondary carrier is a protein. In another preferred embodiment, at least one moiety is conjugated to at least one carrier of the construct. The immunogenicity of the conjugated moiety is greater than the unconjugated moieties.

Another aspect of the invention relates to a vaccine comprising at least one of the dual carrier immunogenic constructs and a pharmaceutically acceptable carrier. A still further aspect of the invention relates to a method of treating a patient by administering an immunostimulatory amount of the vaccine. In another aspect of the invention, the dual carrier immunogenic construct is administered with an adjuvant familiar to those in the art, such as aluminum salts which have been approved for human use.

Another aspect of the invention relates to a method of preparing antibodies by immunizing a host with the vaccine so that antibodies directed against immunogens are produced and then isolating the antibodies or B cells that can be used to produce monoclonal antibodies. In a further aspect, the invention relates to an immunotherapeutic composition comprising such antibodies. In a still further aspect, the invention relates to a method of treating patients by administering a therapeutically effective amount of the immunotherapeutic composition. In another aspect, the invention relates to a diagnostic or research reagent comprising such antibodies.

The accompanying drawings, which are incorporated in and constitute a part of this specification, illustrate several exemplary embodiments of the invention and, together with the description, serve to explain the principles of the invention.

The above and various other objects, features and many of the attendant advantages of the invention will be better understood upon a reading of the following detailed description when considered in connection with the accompanying drawings.

### V. BRIEF DESCRIPTION OF THE DRAWINGS

- Fig. 1: Illustrative depiction of the two classical types of antigens based on their T cell requirements. T-independent antigens (and haptenated TI antigens) stimulate responses in the absence of T cells, and T-dependent antigens (and haptenated TD antigens) require T cell participation for elicitation of optimal antibody responses.
- Fig. 2: Schematic drawing of one embodiment of dual carrier immunogenic construct:
- the primary carrier is a high molecular weight polymer (HMWP), for example Dextran (Dex);
- the secondary carrier, conjugated to Dex, can be any substance that stimulates high levels of T cell activation;
- the hapten is any low molecular weight molecule, such as an oligosaccharide, polysaccharide, peptide, drug, etc., which is conjugated to the secondary carrier.

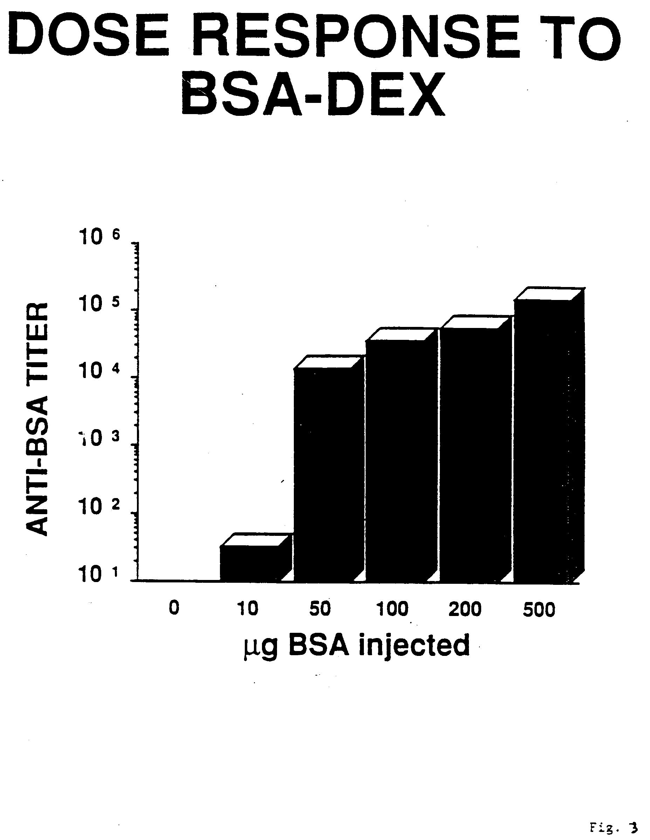

- Fig. 3: Graphic representation of the dose response to BSA-Dex conjugate. Serum IgG1 antibody titers to bovine serum albumin (BSA) were measured in mice immunized intravenously with BSA-Dex in PBS conjugates at doses ranging from 10-500 µg/mouse. Mice were bled 14 days after immunization and antibody titers determined by ELISA. Unconjugated BSA is not immunogenic in mice. This figure shows that conjugation to Dex converts BSA into a highly potent immunogen.

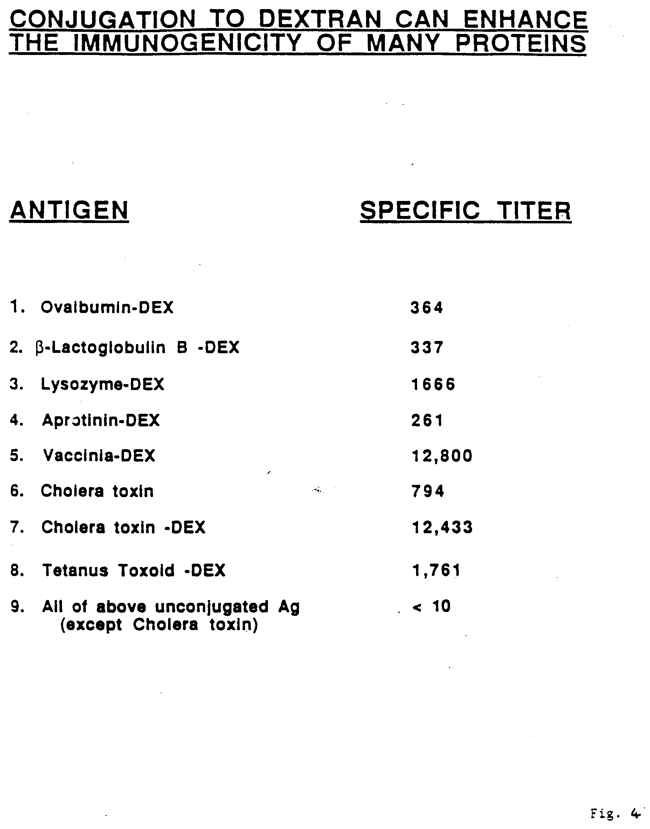

- Fig. 4: Chart depicting proteins of various sizes that were conjugated to Dex and injected intravenously (IV) into mice at the indicated doses. Serum IgG1 antibody titers were determined by ELISA. In contrast to the conjugated proteins, immunization of mice with antigens not coupled to dextran gave no detectable antibody formation (titers were less than 10) except for the cholera toxin immunization which gave detectable titers, albeit significantly less than the Dex conjugate. This figure shows that conjugation of different proteins to Dex converts them into effective immunogens.

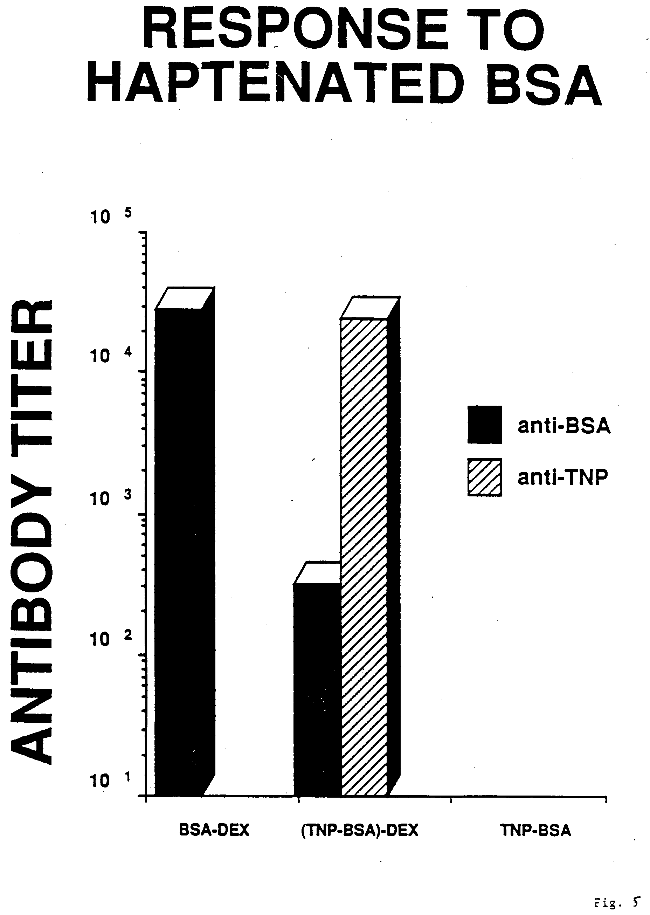

- Fig. 5: Graphic representation of the response to haptenated BSA. Mice were immunized with trinitrophenylated BSA (TNP-BSA) (50 µg) or with TNP-BSA conjugated to Dex (TNP-BSA Dex) (50 µg) and were bled 21 days later. Anti-TNP titters were measured by ELlSA. This figure demonstrates that a good antibody response to both BSA and TNP is elicited by using Dex as the primary carrier and BSA as the secondary carrier. Thus, the conjugation of TNP to the secondary protein carrier molecule coupled with the conjugation of this complex to the primary carrier converted TNP into an effective immunogen.

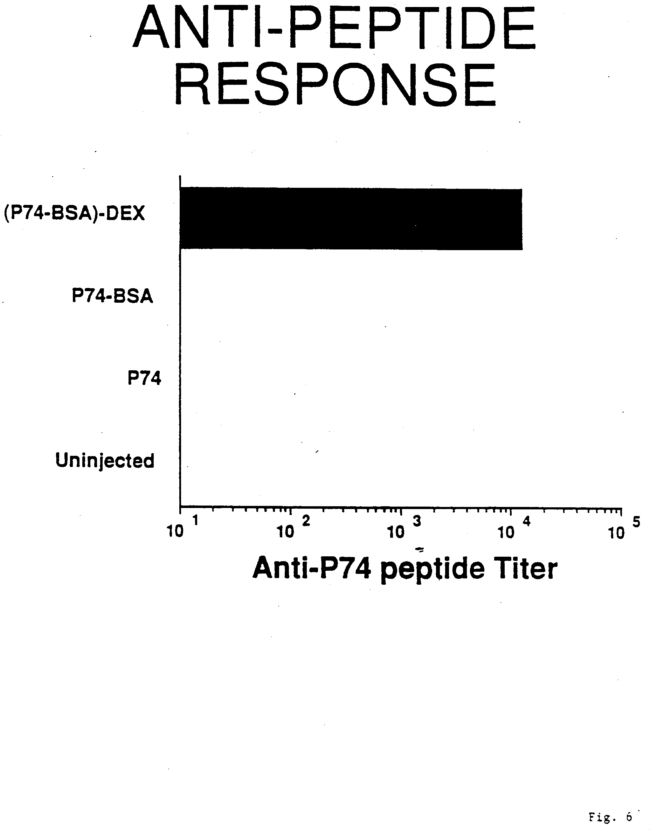

- Fig. 6: Graphic representation of the anti-peptide response. This study supports the previous study shown in Fig. 5. It shows that the conjugation of the malarial derived peptide P74 to the secondary carrier, BSA, and the conjugation of this complex to the Dex primary carrier, converts a non-immunogenic peptide (P74) into an effective immunogen. This is particularly relevant in demonstrating the utility of this construct for synthetic peptides.

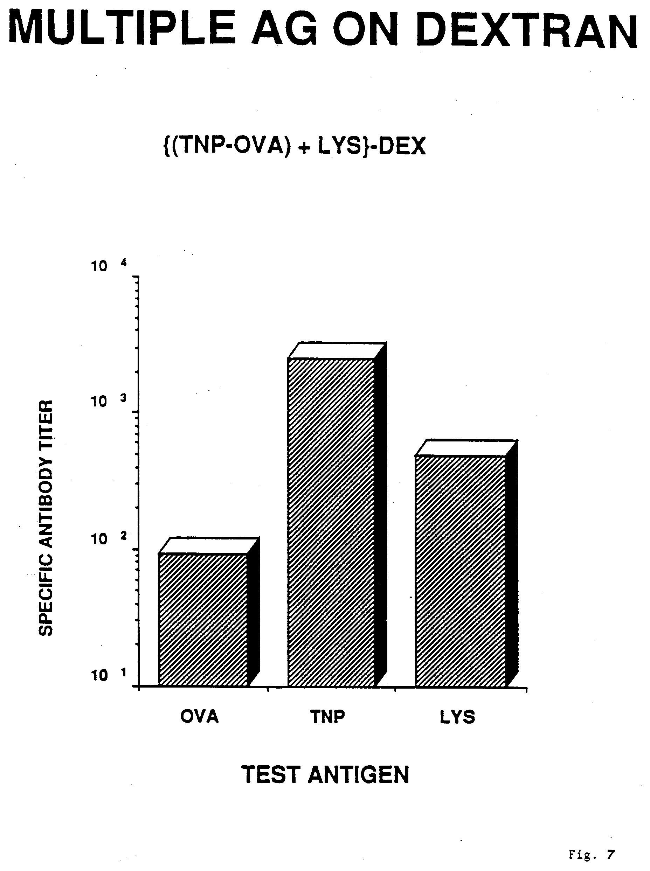

- Fig. 7: Graphic representation of multiple antigens conjugated to dextran. This study evaluates the antibody response to a Dex complex which has been prepared by conjugating three antigenically different molecules to Dex. In this study, TNP coupled with ovalbumin (OVA), plus lysozyme (LYS) were conjugated to Dex and 50 µg of this conjugate was injected IV into mice. Nine days later mice were bled and sera titered by ELISA for anti-OVA, anti-TNP and anti-LYS antibody titers. This study demonstrates that a vaccine preparation can be made by conjugating multiple unrelated antigens onto the primary Dex carrier.

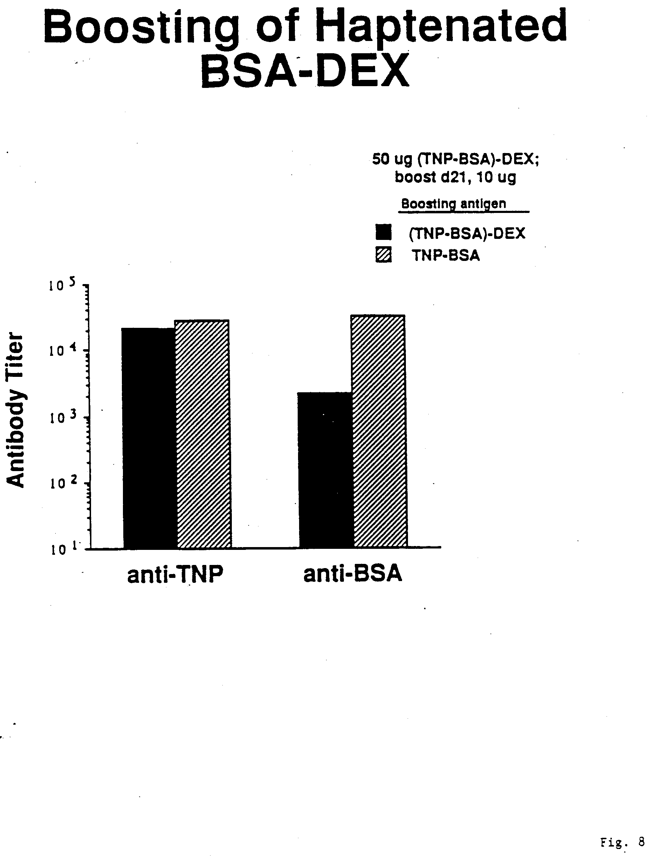

- Fig. 8: Graphic representation of the boosting of haptenated BSA-Dex. Mice were immunized with (TNP-BSA) Dex (50 µg) and then boosted with a second injection of either TNP-BSA alone or with TNP-BSA conjugated to Dex. Mice were bled 14 days later and sera titered for anti-TNP and anti-BSA antibody. This figure shows that once mice are immunized with the Dex conjugate (a construct consisting of both primary and secondary carrier), a good booster response is achieved and that antibody boosting can be achieved as effectively with the unconjugated TNP-BSA (secondary carrier only) as with the complete vaccine construct conjugate. This demonstrates that once a primary response has been stimulated with the conjugate, a large secondary response may be elicited by the unconjugated or natural antigen.

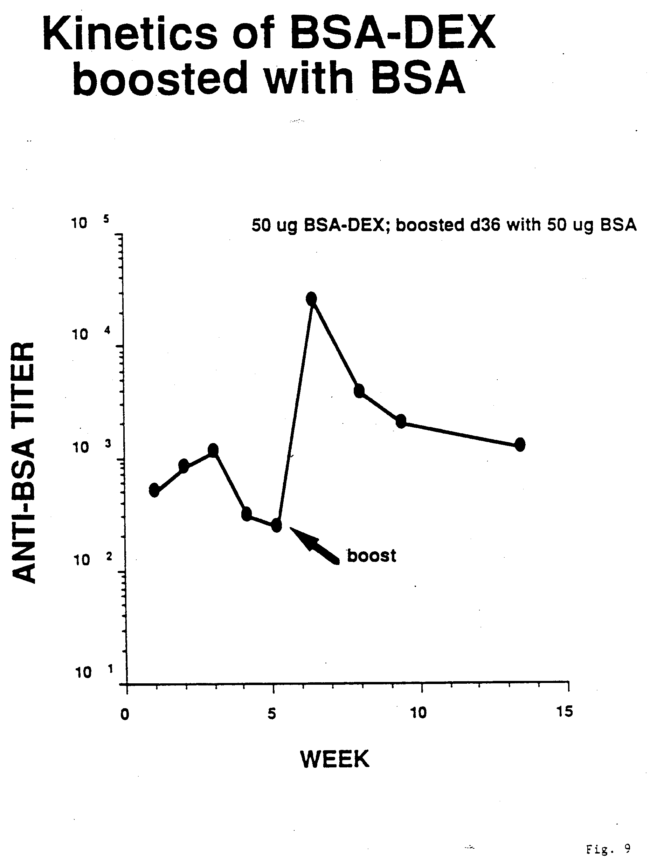

- Fig. 9: Graphic representation of the kinetics of BSA-Dex boosted with BSA. Mice were immunized with 50 µg of BSA-Dex (primary and secondary carrier) and 5 weeks later boosted with 50 µg of BSA (secondary carrier only). Mice were bled at various times and sera titered by ELISA for antibody titers to BSA, the secondary carrier. This figure shows that the secondary antibody responses can be elicited by BSA only, the secondary carrier not conjugated to dextran, and that the response is very long-lived and persists for greater than 11 weeks.

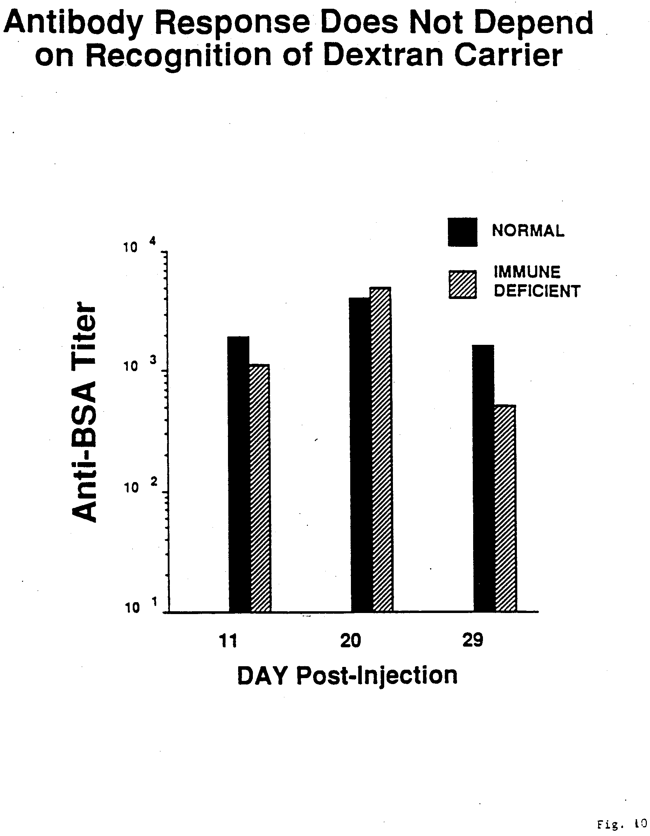

- Fig. 10: Graphic representation of antibody response to the carrier. BSA-Dex conjugates (50 µg) were injected into normal or immune defective mice which are unresponsive to Dex. Mice were bled 11, 20 and 29 days later, and sera anti-BSA titers were measured by ELISA. This study demonstrates that the Dex carrier may simply provide a matrix to present antigen to cells in a multivalent array, that the antibody response to the Dex conjugate does not depend on the ability of mice to mount antibody responses to the dextran carrier, that cells of the immune system need not recognize Dex as an immunogen for it to function as an effective carrier, and importantly that the BSA-Dex conjugate can stimulate responses in immune defective mice.

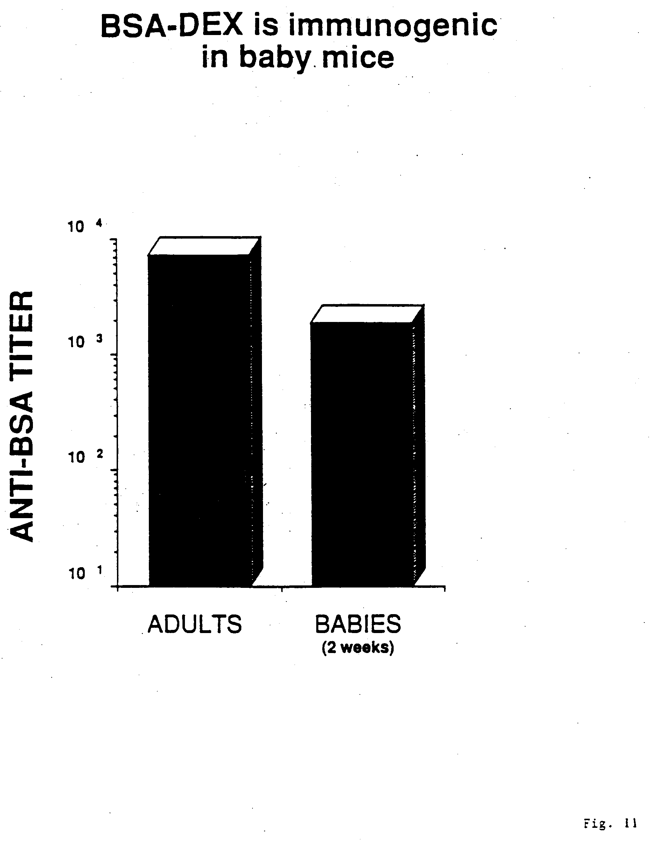

- Fig. 11: Graphic representation of the immunogenicity of BSA-Dex in baby mice. Both 2 week old mice and adult mice were injected intraperitoneally with 50 µg of BSA-Dex and bled 12 days later. Sera anti-BSA titers were measured by ELISA. This study shows that even mice that are immunologically immature can be effectively immunized with this Dex protein-carrier conjugate and elicit good antibody responses to the secondary carrier (BSA).

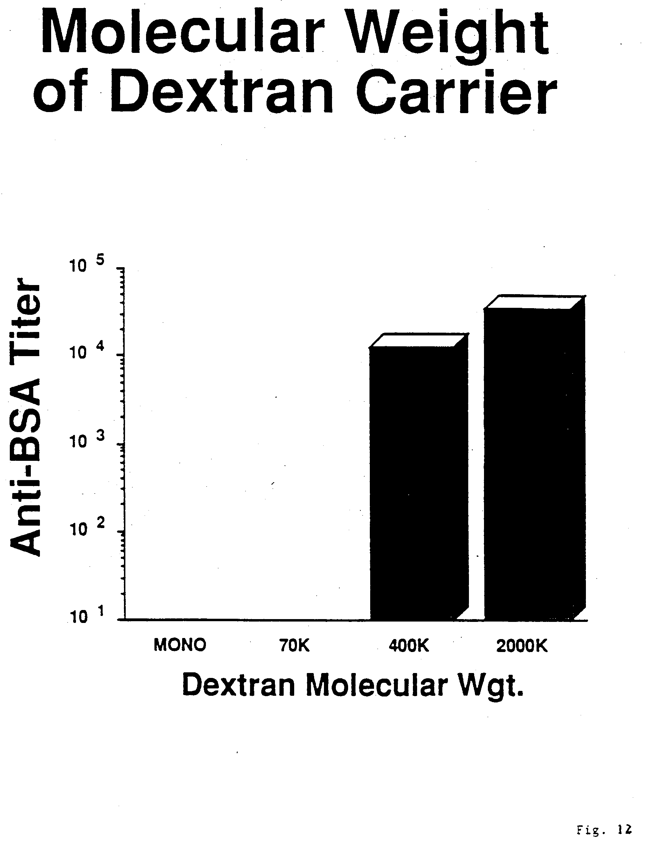

- Fig. 12: Graphic representation of the effect of molecular weight. BSA-Dex conjugates were made using size separated Dex of MW 70K, 400K, or 2000K. Mice were injected IV with 50 µg of the various conjugates and bled 14 days later. Serum antibody titers were determined by ELISA. This figure shows that Dex must be >70KD in size to provide an effective carrier molecule and that, although 400 KD elicits a good response, a larger molecular weight carrier molecule is even more effective.

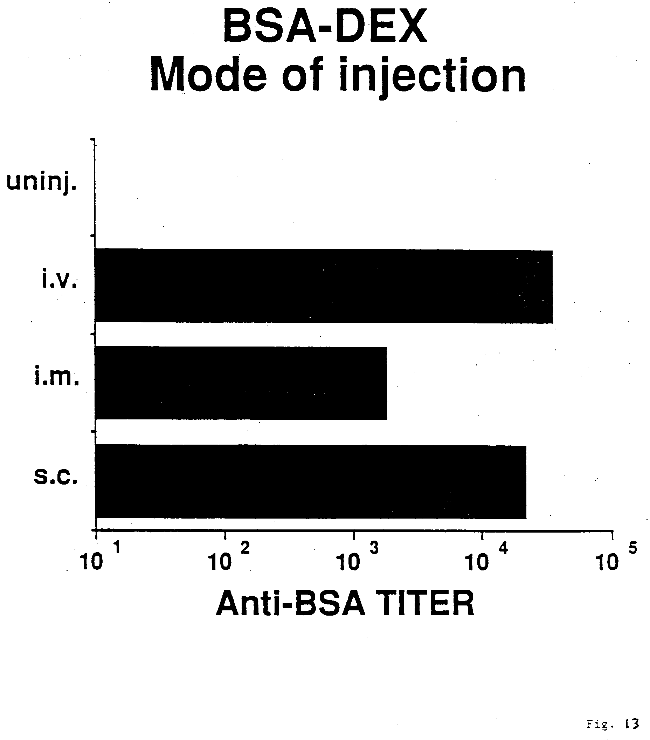

- Fig. 13: Graphic representation of the effect of injection mode. Mice were immunized with BSA-Dex via three different routes: intravenously (IV), subcutaneously (SC), or intramuscularly (IM). Mice were bled 14 days later and sera titers determined by ELISA. This study shows that the Dex protein-carrier conjugate (primary and secondary carrier complex) stimulates comparable responses whether given IV, SC or IM.
- Fig. 14: Schematic drawing of the dual carrier immunogenic construct to which multiple and different secondary carriers (tetanus toxoid, meningococcal outer membrane protein, and viral protein) are conjugated to at least one primary carrier. The secondary carriers may or may not be further haptenated.
- Fig. 15: Graphic representation of the success of the conjugation of pertussis toxin to dextran. The profile is of PT-dex applied to a S400SF gel filtration column equilibrated with saline and azide. Flow rate was 0.2 ml/min., 25 drops/tube. The arrow indicates the position of unconjugated toxoid. OD 280 is a measure of protein and CPM/50 ul refers to the counts per minute present in a 50 ul aliquot, a measure of the amount of tritiated dextran present.

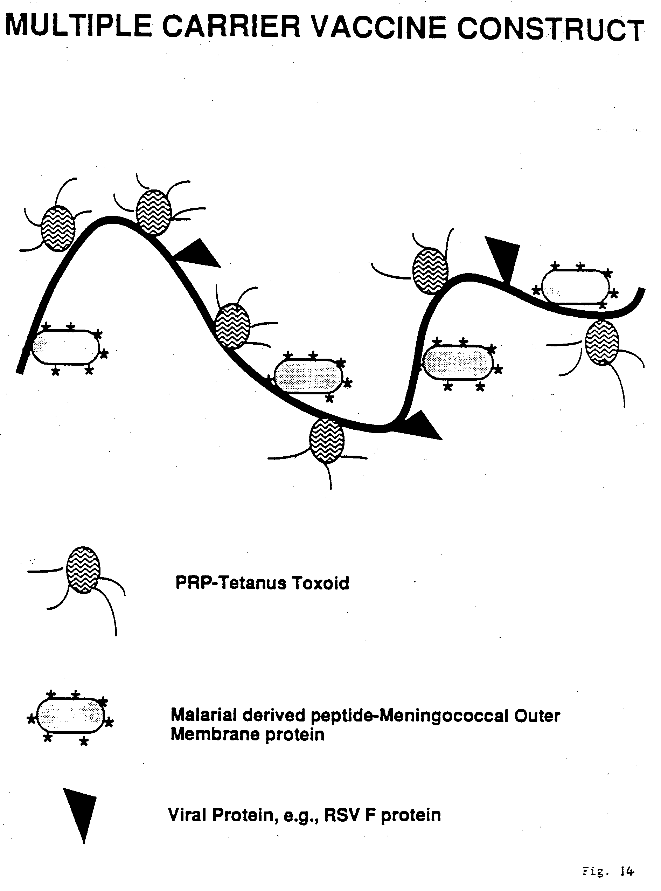

- Fig. 16: Chart depicting the anti-dextran response of mice injected with a single dose of tetanus toxoid-dextran, diptheria toxoid-dextran, or pertussis toxoid-dextran as fluid or absorbed onto the adjuvant aluminum phosphate. The chart shows that the mice generated antibodies, predominantly IgG1, IgG2, and IgM, by two weeks and that the antibodies persisted for at least eight weeks. (See Example 9 for details.)

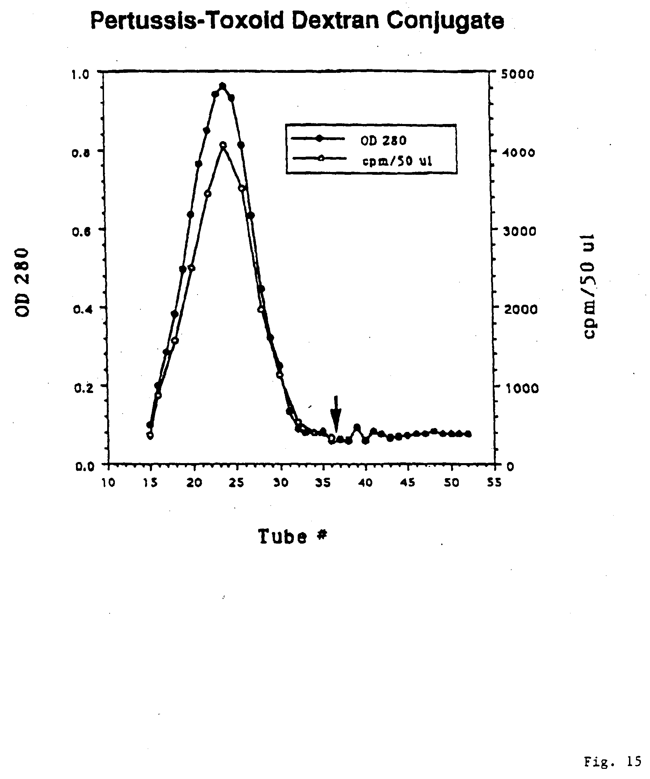

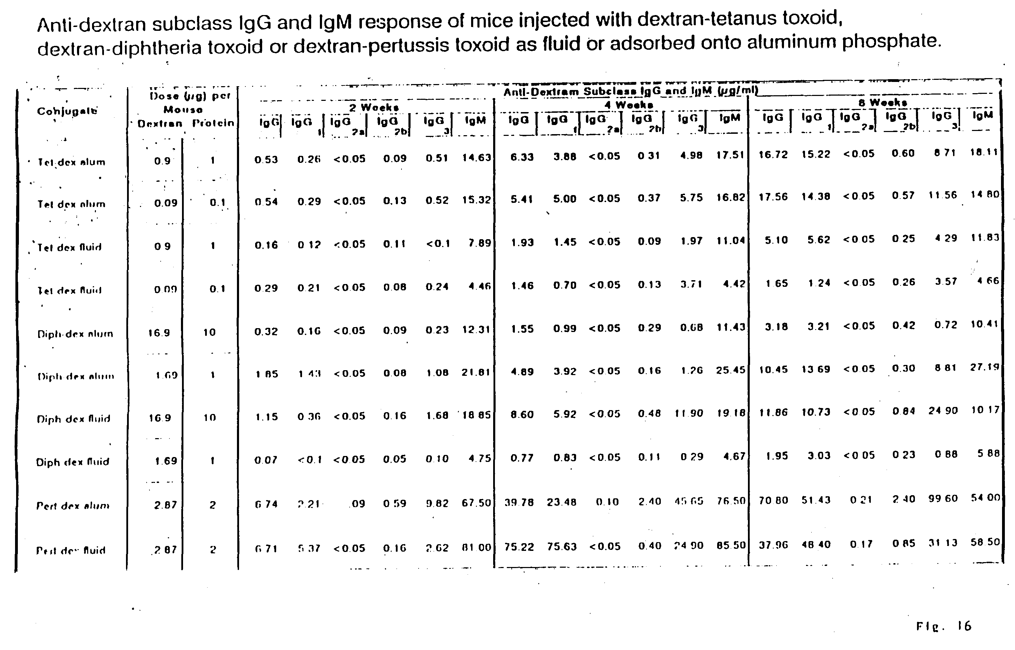

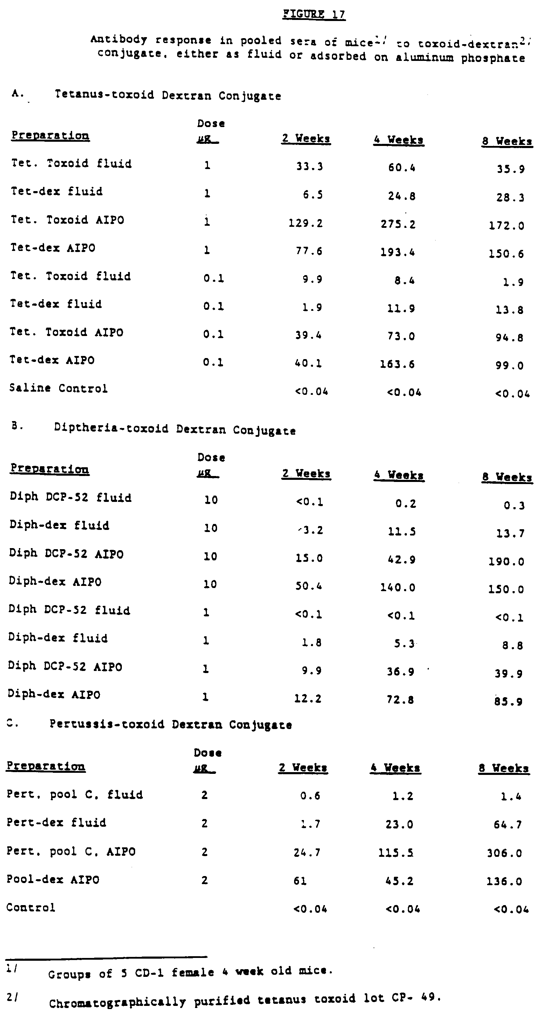

- Fig. 17: Three-part graphic demonstration of the antibody response to the secondary carriers, diptheria toxoid, tetanus toxoid, and pertussis toxoid when conjugated to high molecular weight dextran. As with the anti-dextran antibodies, antibodies against these proteins were elicited by at least two weeks and persisted for at least eight weeks. Together, Figs. 15 and 16 demonstrate the dual and reciprocal enhancement achieved with the conjugates of the invention. (See Example 9 for details.)

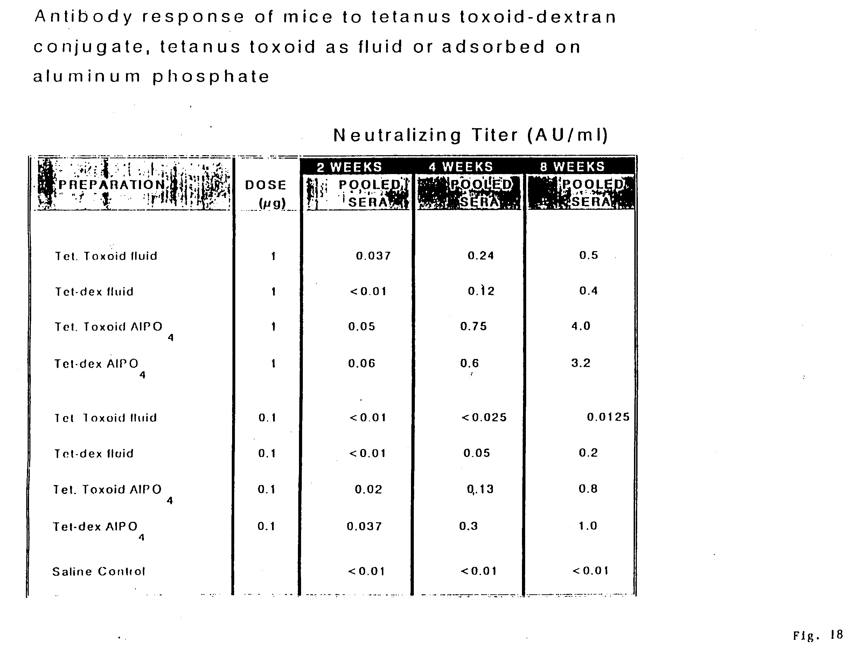

- Fig. 18: Graphic representation of the neutralization affect of the tetanus toxoid, showing that the conjugate generated protective levels of anti-toxin antibodies.

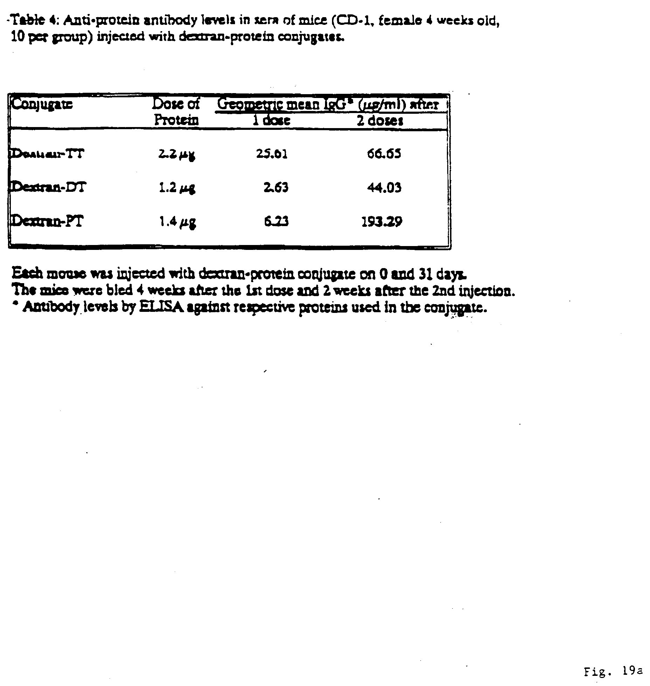

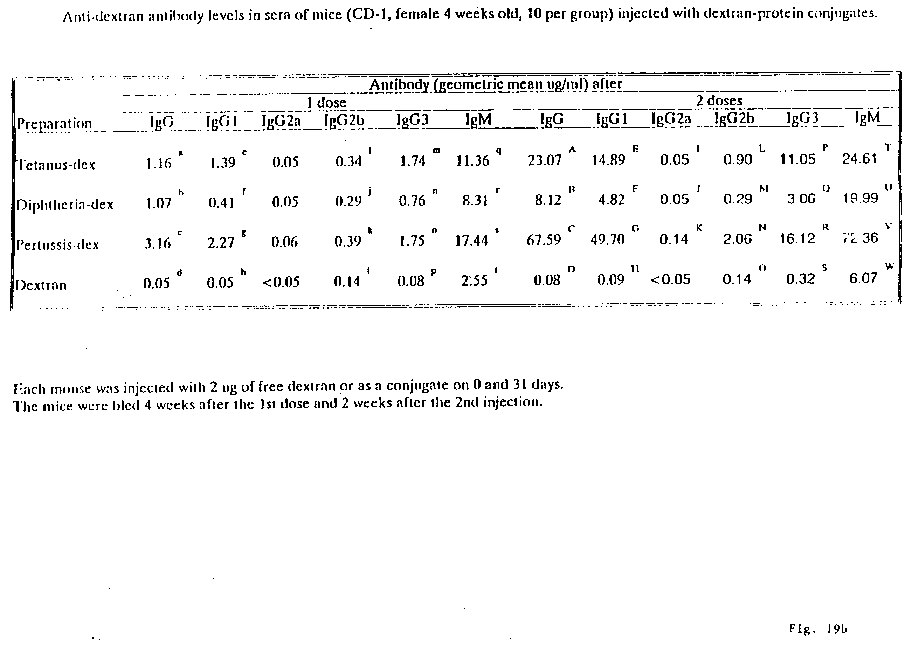

- Fig. 19: Two-part graphic demonstration of the dual and reciprocal immune enhancement achieved with the conjugates of the invention with two doses of conjugate administered according to Fig. 19a. Part (a) sets forth the anti-protein antibody levels induced by the secondary carriers. Part, (b) sets forth the anti-dextran antibody levels categorized by isotype.

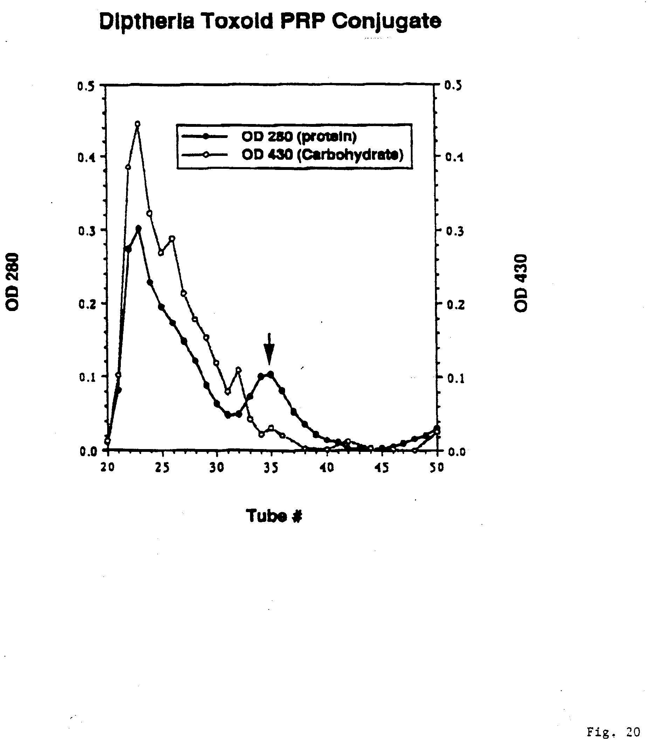

- Fig. 20: Graphic depiction of conjugation of diptheria toxin to PRP. The profile is of DT-PRP applied to a S300SF gel filtration column equilibrated with PBS. Flow rate was 0.25 ml/min., 25 drops/tube. The arrow indicates the position of unconjugated toxoid. OD 280 is a measure of protein and OD 430 refers to the absorbance produced in the resorcinol carbohydrate assay (Dubois, H. et al. Anal. Chem 28:350 (1956)) and is a measure of the relative amount of PRP.

### VI. DETAILED DESCRIPTION OF PREFERRED EMBODIMENTS

Reference will now be made in detail to the presently preferred embodiments of the invention, examples of which are illustrated in the accompanying drawings.

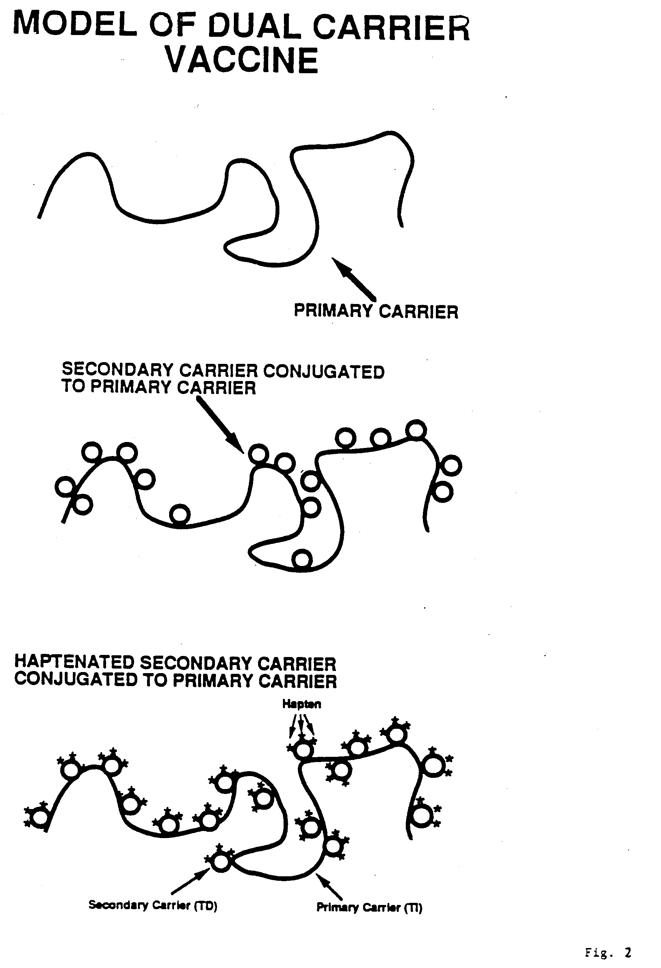

The invention relates to an immunogenic construct made up of at least two carriers, at least one primary carrier that is a large molecular weight molecule of greater than 70 kDa molecular weight and a secondary carrier that is a T-dependent antigen conjugated thereto as represented in Figure 2. A carrier is any substance to which other substances may be attached so that the immunogenicity of the attached substances is enhanced.

In a preferred embodiment, the immunogenicity of the construct is greater than the immunogenicity of at least one carrier alone. Methods of measuring immunogenicity are well known to those in the art and primarily include measurement of serum antibody including measurement of amount, avidity, and isotype distribution at various times after injection of the construct. Greater immunogenicity may be reflected by a higher titer and/or persistence of antibody response. Immunogenicity may also be measured by the ability to induce protection to challenge with noxious substances or organisms. Immunogenicity may also be measured by the ability to immunize neonatal and/or immune defective mice. Immunogenicity may be measured in the patient population to be treated or in a population that mimics the immune response of the patient population.

As a result of the contributions of both types of carriers, the dual carrier construct is an extremely potent activator of T cell help via mechanisms such as enhanced antigen presentation by B cells, macrophages or other antigen presenting cells. Such a construct will elicit very rapid and long lived antibody formation in adults, children, and those with immature or immunodeficient immune systems.

The construct of the invention is preferably water soluble or may be maintained in aqueous media. The solubility may derive from the use of solubilizing reagents during the synthesis of the construct. In addition, the construct of the invention may be maintained in aqueous media by the use of solubilizing reagents.

The process of synthesizing the construct of the invention allows one to advantageously control the physical and chemical properties of the final product. The properties that may be controlled include modifying the charge on primary and secondary carriers (an advantage in light of evidence that cationized proteins may be more immunogenic), varying the size of the construct by varying the size of the primary carriers, selecting the degree of crosslinking of the construct (to obtain variations of size and half-life in the circulation), selecting the number of copies of secondary carriers conjugated to primary carriers, and targeting to selected cell populations (such as to macrophages to enhance antigen presentation).

The immune response to the construct of the invention may be further enhanced by the addition of immunomodulators and/or cell targeting moieties. These entities include, for example, (1) detoxified lipopolysaccharides or derivatives, (2) muramyl dipeptides, (3) carbohydrates, lipids, and peptides that may interact with cell surface determinants to target the construct to immunologically relevant cells, (4) interleukins, and (5) antibodies that may interact with cell surface components.

As represented in Figure 2, the construct of the invention is made up of at least one primary carrier that provides a large backbone matrix to which one or more copies of secondary carrier may be conjugated. In addition, as will be discussed below, one or more primary or secondary carriers may be further conjugated to moieties. Methods of conjugation are well known to those of ordinary skill in the art, and include the heteroligation techniques of Brunswick M., et al., J. Immunol. 140:3364 (1988) and Mongini P.K., et al., J. Immunol. 148:3892 (1992), both specifically incorporated herein by reference. See also Wong, S.S. Chemistry of Protein Conjugates and Crosslinking CRC Press, Boston (1991), specifically incorporated herein by reference. We have found, however, that these methods, though well known in the art for conjugating proteins, may require some modification to optimize their application to the synthesis of conjugate vaccines.

As those in the art may appreciate, the antigens used in the preparation of conjugate vaccines are valuable. Therefore, it is desirable to use chemical procedures which give high yields of product and which minimally modify important epitopes. Many protocols for conjugating proteins to carbohydrate use carbodiimides to form amide linkages (Cruse J.M., Lewis R.E. Jr. ed., Conjugate vaccines in Contributions to Microbiology and Immunology, vol. 10, 1989). This reagent is nonselective, causes extensive modification and potentially undesirable polymerization of the protein. Yields are often low. Other procedures result in disulfide linkages, which are less stable than the thio-ether bond (Cruse J.M., Lewis R.E. Jr. ed., Conjugate vaccines in Contributions to Microbiology and Immunology, vol. 10, 1989).

In contrast, the chemistry of the invention likely avoids these difficulties. The elements of a preferred protocol are (1) derivatization of the secondary carrier (such as a toxin protein) with thiol, (2) derivatization of the primary carrier polymer with thiol reactive reagents; e.g., maleimide, iodoacetate, (3) formation of a thio-ether linkage and (4) separation of conjugate from unconjugated polymer and protein.

Derivatization of the protein and carbohydrate is normally followed by a column desalting step and concentration. An alternative, which has been successfully used, is to perform all the steps, or preferable steps 1-3, with an ultrafiltration device (e.g., Amicon pressure filtration) using suitable molecular weight cut off filters. This minimizes handling and transfers.

By controlling the number of reagent groups and the concentrations of the components and other details, this chemistry allows relatively facile control of the degree of interchain crosslinking, degree of polymerization and aggregration to result in a rapid method achieving high yield and minimal modification of key epitopes.

As the skilled artisan would recognize, the specific conjugation methodology may vary depending on the individual primary and secondary carriers but, given the teaching herein and the knowledge of those in the art, any other methodologies or necessary modifications to the methodologies presented herein are within the skill of those in the art. To assist the skilled person, however, representative conjugations techniques for particular primary carriers are set forth herein. These techniques are directed to the primary carriers dextran (see section entitled "Method") and haemophilus influenzae polysaccharide (PRP) (see Example 11), although the techniques may be used with the other primary carriers described below. Other chemical techniques may be used with dextran or PRP and other primary carriers, as known to those skilled in the art.

The conjugation of carriers within this invention may provide minimal disruption of critical epitopes on the carriers, since protein conjugation to a primary carrier, such as dextran, involves minimal alterations to the dextran. In addition, a primary carrier within the invention may also include functional groups or, alternatively, may be chemically manipulated to bear functional groups. As is done with dextran and PRP, the presence of functional groups may facilitate covalent conjugation of a primary carrier to one or more secondary carriers. Such functional groups include, but are not limited to, amino groups, carboxyl groups, aldehydes, hydrazides, epoxides, and thiols.

The large backbone of each primary carrier provides an ideal matrix for many different secondary carriers so that one vaccine could contain multiple antigenic specificities. Moreover, the primary carrier stimulates antibody production by presenting numerous copies of secondary carriers at a relatively high antigenic density to both B and T cells. It may also provide other advantages including targeting of the construct to macrophages or other cell types to enable enhanced antigen processing.

In a preferred embodiment, the molecular weight of at least one primary carrier ranges from greater than 70,000 to 2,000,000 kDa and above. As set forth in Figure 12, a more preferred molecular weight is 400,000 kDa and above, and an even more preferred molecular weight is 2,000,000 kDa. The conjugation of the primary carrier to at least one secondary carrier may result in crosslinking of the primary carrier. Such crosslinking may permit the use of lower molecular weight carriers (such as 70,000 kDa) so long as the final construct is of a higher molecular weight. Based on the teachings contained herein together with the ordinary skill in the art, the skilled artisan will know how to select the optimum molecular weight for the particular construct desired.

In another preferred embodiment, at least one primary carrier is a T-independent antigen, thereby combining the advantages of T-independent and T-dependent antigens. Such a carrier that can itself directly and potently activate B cells and can serve as a large but relatively nondegradable backbone to carry many secondary carriers. As detailed below, however, the invention may be practiced, however, with a primary carrier that is not immunogenic by itself.

A primary carrier may be naturally occurring, a semisynthetic or a totally synthetic large molecular weight molecule. In a preferred embodiment, at least one primary carrier is a polymer selected from the group consisting of dextran, carboxymethyl cellulose, agarose, ficoll, polyacrylamide, naturally occuring polysaccharides, and combinations thereof.

In one preferred embodiment, the primary carrier is a dextran. As used herein, dextran ("dex") refers to a polysaccharide composed of a single sugar and may be obtained from any number of sources, including Pharmacia. Ficoll, an example of a semi-synthetic polymer, is an inert synthetic non-ionized high molecular weight polymer. Synthetic polymers include polyacrylamide (a water-soluble high molecular weight polymer of acrylic resin), poly (lactide-co-glycolide), polyvinyl alcohol, partially hydrolyzed polyvinyl acetate, and polyvinylpyrrolidine. In another preferred embodiment, the primary carrier is a microbial polysaccharide. In still another embodiment, the microbial polysaccharides are derived from pathogenic microorganisms. In yet a further preferred embodiment, the primary carrier is selected from the group consisting of Pneumococcal capsular polysaccharides (including type III), Group B Streptococcus serotypes, Pseudomonas aeruginosa mucoexopolysaccharides, P. aeruginosa capsular polysaccharides (including strain fisher type 1), Haemophilus influenzae polysaccharide (including PRP), and combinations thereof. In a still further preferred embodiment, the primary carrier is haemophilus influenzae polysaccharide (PRP).

The secondary carrier of this construct also provides specific advantages for eliciting good antibody responses. As a T-dependent antigen, the secondary carrier can activate and recruit T cells and thereby augment T cell dependent antibody production. The secondary carrier need not, however, be strongly immunogenic by itself, although strongly immunogenic carriers are within the scope of this invention. Coupling of multiple copies of the secondary carrier to the primary carrier significantly augments antibody production against the secondary carrier even in the absence of adjuvants.

In a preferred embodiment, the secondary carrier is a protein, a peptide, a T cell adjuvant or any other compound capable of activating and recruiting T cell help. The secondary carrier may be selected from a group consisting of but not limited to viral, bacterial, parasitic, animal and fungal constituents. In a more preferred embodiment, the secondary carrier is a protein such as albumin (such as bovine serum albumin), tetanus toxoid, diphtheria toxoid, pertussis toxoid or bacterial outer membrane protein, all of which may be obtained from biochemical or pharmaceutical supply companies or prepared by standard methodology (Cruse, J.M. and Lewis, R.E, Jr. (eds.) Conjugate Vaccines in Contributions to Microbiology and Immunology vol. 10 (1989)), specifically incorporated herein by reference). Other components that could function as secondary carriers would be known to those of ordinary skill in the art of immunology.

The secondary carriers of the invention are capable of being conjugated to at least one primary carrier. The secondary carriers may either contain functional groups that can react with the primary carriers or the secondary carriers may be chemically manipulated to be capable of reacting with the primary carriers discussed above.

As described above, numerous copies of specific secondary carriers as well as a variety of secondary carriers may be conjugated to the primary carrier. Coupling of multiple copies of the secondary carrier to the primary carrier significantly augments antibody production to the secondary carrier.

The secondary carriers of the invention are preferably water soluble, whether conjugated or unconjugated or whether coupled to the immunogens discussed below.

In another embodiment, moieties may be further conjugated to one or more of the primary and/or secondary carriers, as represented in Figure 2. Such conjugation promotes enhanced antibody responses to the moiety. Techniques to conjugate such moieties to the primary and/or secondary carriers are well known to those skilled in the art, and include, in part, coupling through available functional groups (such as amino, carboxyl, thio and aldehyde groups). See S.S. Wong, Chemistry of Protein Conjugate and Crosslinking CRC Press (1991), and Brenkeley et al., Brief Survey of Methods for Preparing Protein Conjugates With Dyes, Haptens and Cross-Linking Agents, Bioconjugate Chemistry 3 #1 (Jan. 1992), specifically incorporated herein by reference. The haptens of the invention may first be conjugated to the secondary carrier (which may be the T-dependent component) and then coupled to the primary carrier (which may be the T-independent component) which are thereafter coupled. Alternatively, the secondary carrier may be first conjugated to the primary carrier and then haptens conjugated to this construct. This second method may aid in minimizing the modification of certain haptens such as peptide haptens containing amino groups.

As used herein, moiety is any substance that is able to stimulate the immune system either by itself or once coupled. Moieties include haptens, antigens, or combinations thereof. Haptens refer to small molecules, such as chemicals, dust, and allergens, that by themselves are not able to elicit an antibody response, but can once coupled to a carrier. An antigen is any molecule that, under the right circumstances, can induce the formation of antibodies. These haptens and antigens may derive from but are not limited to bacteria, rickettsiae, fungi, viruses, parasites, drugs, or chemicals. They may include, for example, small molecules such as peptides, saccharides, oligosaccharides (for example the polyribosyl-ribitol-phosphate of H. influenzae), toxins, endotoxin, etc.

In another embodiment, the invention relates to vaccines that are made up of the dual carrier immunogenic construct together with a pharmaceutically acceptable carrier. Such vaccines will contain an effective therapeutic amount of the dual carrier immunogenic construct together with a suitable amount of carrier so as to provide the form for proper administration to the patient.

Pharmaceutically acceptable carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical carriers are described in Martin, E.W., Remington's Pharmaceutical Sciences, specifically incorporated herein by reference.

The vaccines that may be constructed from the dual carrier immunogenic construct of the invention may include, but are not limited to, the vaccines set forth in Chart 1.

| Chart 1 |
|---|
| Diphtheria vaccine |
| Pertussis (subunit) vaccine |
| Tetanus vaccine |
| H. influenzae, type b (polyribose phosphate) |
| S. pneumoniae, all serotypes |
| E. coli, endotoxin or J5 antigen (LPS, Lipid A and Gentabiose) |
| E. coli, O polysaccharides (serotype specific) |
| Klebsiella, polysaccharides (serotype specific) |
| S. aureus, types 5 and 8 (serotype specific and common protective antigens) |
| S. epidermidis, serotype polysaccharide I, II and III (and common protective antigens) |
| N. meningiditis, serotype specific or protein antigens |
| Polio vaccine |
| Mumps, measles, rubella vaccine |
| Respiratory syncytial Virus |
| Rabies |
| Hepatitis A, B, C, and others |
| Human immunodeficiency virus I and II (GP120, GP41, GP160, p24, others) |
| Herpes simplex types 1 and 2 |
| CMV |
| EBV |
| Varicella/Zoster |
| Malaria |
| Tuberculosis |
| Candida albicans, other candida |
| Pneumocystis carinii |
| Mycoplasma |
| Influenzae virus A and B |
| Adenovirus |
| Group A streptococcus |
| Group B streptococcus, serotypes, Ia, Ib, II and III |
| Pseudomonas aervinosa (serotype specific) |
| Rhinovirus |
| Parainfluenzae, types 1, 2 and 3 |
| Coronaviruses |
| Salmonella |
| Shigella |
| Rotavirus |
| Enteroviruses |
| Chlamydia trachomatis & pneumoniae (TWAR) |

The invention also relates to the treatment of a patient by administration of an immunostimulatory amount of the vaccine. Patient refers to any subject for whom the treatment may be beneficial and includes mammals, especially humans, horses, cows, dogs, and cats as well as other animals, such as chickens. An immunostimulatory amount refers to that amount of vaccine that is able to stimulate the immune response of the patient for the prevention, amelioration, or treatment of diseases. The vaccine of the invention may be administered by any route, but is preferably administered by intravenous, intramuscular, and subcutaneous injections.

The invention also relates to a method of preparing an immunotherapeutic agent against infections caused by bacteria, viruses, parasites, fungi, or chemicals by immunizing a host with the vaccine described above so that the, donor produces antibodies directed against the vaccine. Antibodies would be isolated or B cells may be obtained to later fuse with myeloma cells to make monoclonal antibodies. The method of making monoclonal antibodies is well known in the art, Kohler and Milstein Nature 256:495 (1975), specifically incorporated herein by reference, and needs no further description here. As used herein, immunotherapeutic agent refers to a composition of antibodies that are directed against specific immunogens for use in passive treatment of patients. A plasma donor is any subject that is injected with a vaccine for the production of antibodies against the immunogens contained in the vaccine.

The invention also relates to a method of treating a patient by the administration of a protective amount of the immunotherapeutic agent. Such a treatment is passive in that it does not call on the patient to produce antibodies against an immunogen, but rather uses antibodies produced by the plasma donor against the immunogen. The amount of therapeutic antibodies is protective if it presents a sufficient number of antibodies that can prevent, ameliorate, or treat the disease caused by the immunogen. Such an amount may be determined by those of ordinary skill in the art and varies based on the characteristics of the patient and the disease profile.

The invention also relates to a method of producing a diagnostic and/or research reagent to detect agents that are characteristic of diseases caused by, for example, bacteria, viruses, fungi, parasites or chemicals by immunizing a host with a vaccine described above so that the host produces antibodies (or B cells) against the agents. The antibodies and/or B cells may be isolated as described above. As used herein, diagnostic reagent refers to a composition of antibodies (polyclonal or monoclonal) that may be used to detect agents that are characteristic of diseases. As used herein, research reagent refers to a composition of antibodies (polyclonal or monoclonal) that may be used in the laboratory.

The herein offered examples provide methods for illustrating; without any implied limitation, the practice of this invention in the production of vaccines to produce antibodies either monoclonal or polyclonal, which could be employed for active or passive prophylaxis or therapy, and for diagnostic, or research purposes.

The profile of the representative experiments have been chosen to illustrate methods for producing a novel dual carrier immunogenic construct and the concepts of function.

### METHODS

I. Mice (DBA/2J) were used at 8 weeks old unless otherwise noted and were immunized with 0.1 ml and a saline solution of various antigens intravenously, subcutaneously, or intramuscularly. In all experiments 5 mice per group were used. Bleeding was by tail vein.
II. Amino ethyl carbamyl Dex (AECM Dex)
   Amino-ethyl carbamyl dextran (AECM Dex) was prepared essentially as described by Brunswick et al., J. Immunol. 140:3363 (1988), specifically incorporated herein by reference. AECM T2000 dextran (Pharmacia) was passed over a gel permeation column (CL2B column 2.5 x 105 cm). Material from the first third of the column was pooled and determined to have an average molecular weight of 2,000,000 daltons. This material is now referred to as HMW AECM Dextran. AECM dextrans were also prepared from T70 and T500 dextran (Pharmacia) and fractionated on CL6B and CL4B columns, respectively. A center cut was taken, concentrated and rerun to obtain a relatively homogenous preparation. Trinitrobenzene sulfonic acid (TNBS) was used to determine the average number of amino groups per dextran molecule. High molecular weight dextran preparations had from 150 to 200 amino groups per 2,000,000 daltons. The AECM T500 preparation had an average of 150 amino groups per 400,000 daltons and the AECM T70 preparation had an average of 35 amino groups per 70,000 daltons. In order to facilitate the determination of dextran concentrations, AECM dextran was routinely radiolabelled by reaction with a small amount of N-succinimidy [3H-2,3] propionate (Amersham) [3H-NSP]. Approximately one in 10,000 dextran molecules were modified. (3H-AECM HMW Dex) was lightly trinitrophenylated as follows: 50 ul of 0.01 M freshly prepared TNBS in O.O1M sodium borate pH 9.3 was added to a stirred solution of 20 mg of HMW AECM-Dex in 2 ml HEPES buffer [0.15 M HEPES, 2 mM EDTA, 0.02% azide, pH 7.5] plus 0.5 ml 0.1 M sodium borate buffer pH 9.3. After 2 hours in the dark at room temperature, reagents were removed on P6DG desalting column (BioRad) and the labeled dextran concentrated by ultrafiltration using a Centricon 30 (Amicon). Using a molar extinction coefficient of 11,000 at 366 nm, the ratio of TNP to HMW dextran was determined to be 40:1. This left approximately 100-150 free amino groups for further reaction.
   Modifications of this method may be useful for other primary carriers. For example, Pseudomonas aeruginosa mucoexopolysaccharide (MEP), which is a 500 kDa molecule containing carboxyl groups, may first be partially derivatized with ethylene diamine and a water soluble carbodiimide. In contrast, P. aeruginosa fisher type 1 polysaccharide which contains amino groups, may respond well with the standard protocol but may be assisted by the presence of high salt.
III. Conjugation of proteins to AECM Dex

Proteins were conjugated to the AECM dextran using heteroligation techniques (Brunswick M, et al., J. Immunol. 140:3364, 1988, specifically incorporated herein by reference). Protein was acetylthiolated and the dextran iodoacetylated with the reagent SIAP (Brunswick M, et al., J. Immunol. 140:3364, 1988, specifically incorporated herein by reference) but other reagents such as iodoacetic acid N-hydroxy succinimide ester (IANHS) could be used.

Proteins were typically reacted with 4-8 fold molar excess of the reagent SATA (Calbiochem) for 1-2 hours. The activated dextrans and protein were each desalted into Acetate buffer (10 mM NaAcetate, 0.1 M NaCl, 2 mM EDTA, 0.02% SodiumAzide, pH 5.0) to remove excess reagent and concentrated using a Centricon 30. Protein and dextran were typically mixed at molar ratios of 30-60:1, the pH raised to 7.5 with concentrated HEPES buffer + hydroxylamine. The final concentrations were 75 mM HEPES, 2mM EDTA, 0.02% azide and 50 mM hydroxylamine. After an overnight reaction at 4°C, the conjugate was treated with 0.2 mM mercaptoethanol for 1 hour (to consume any remaining iodoacetyl groups) followed by 10 mM iodoacetamide (to consume all thiol groups), further concentrated if necessary (by, for example, a centrifugation device or pressure ultra filtration), and passed over a 1 x 58 cm gel filtration column, equilibrated with PBS, containing S200SF, S300SF or S400SF (Pharmacia), depending on the molecular weight of the protein. The radioactive void column peak was pooled and concentrated if necessary. Solutions were sterilized by passage through a Millex GV or HV filter (Millipore).

P74 peptide (CNIGKVPNVQDQNK, single letter amino acid code), was conjugated to BSA as follows. 11.6 mg BSA (Pentex) in 400 ul HEPES buffer was made 10 mM in iodoacetamide. This was to block any native thiol groups which might react with the heterobifunctional reagent and cause polymerization. After a 10 minute incubation, the protein was iodoacetylated by adding a 12-fold molar excess of SIAP. After one hour, the solution was desalted into acetate buffer and concentrated to 39 mg/ml.

The thiol peptide was radiolabeled so that the amount to peptide conjugated to BSA could be estimated. The peptide was dissolved in HEPES buffer and a 1.5 fold molar excess of Ellman's reagent was added to block the thiol. After 30 minutes, a 10 fold molar excess of N-ethyl maleimide was added to consume thiols of the half-Ellman's reagent released. 1 hour later the thiol protected peptide was radiolabeled with N-succinimidyl [³H-2,3]-propionate (³H-NSP) (Amersham). Just before conjugation, the peptide solution was made 50 mM dithiothreitol and all reagents removed on a 1 x 38 cm G-10 column (Pharmacia). The radioactive peak running in the void volume was pooled. The specific activity of the peptide was about 2.5 x 10¹¹ cpm/mole. The radiolabeled thiol peptide was added to 4.5 mg iodoacetylated BSA at a molar ratio of 15:1 and the pH raised to 7.5 by the addition of 5x HEPES buffer. The final volume was 1.2 ml. After an overnight reaction, the solution was treated with 0.2 mM mercaptoethanol for 1 hour and the unreacted peptide removed on a 1 x 15 cm P6DG column equilibrated with PBS. The final peptide BSA ratio was determined to be 6:1. This peptide-protein conjugate was then coupled to dextran as described for BSA.

B-lactoglobulin B, aprotinin, ovalbumin (OVA) and lysozyme were obtained from Sigma. Bovine serum albumin (BSA) was from Pentex or Amresco (Biotech grade). Vaccinia protein was the generous gift of Dr. Isabella Quarkyi (Georgetown University Medical School).

TNP-OVA and TNP-BSA was prepared by adding a 4-12 fold molar excess (from a 0.25 M stock) of TNBS in O.1M NaBorate, 0.2 M NaCl, 0.02% NaAzide, pH9.1) to a 50 mg/ml solution of OVA or BSA respectively in the same buffer. After an overnight reaction at 4°C, the protein was dialyzed into HEPES buffer. The ratio was determined from the OD at 366 nm and correcting the hapten contribution at 280 nm. (OD280 = .32 x OD 366).
IV. Serum IgGl, and IgM anti-TNP titers were determined by and ELISA. This assay was performed similarly to our previously described assay, except in this case we used alkaline phosphate conjugated antibodies and microtiter wells were coated for 2 hours with 10 µg/ml of TNP Ficoll for measuring anti TNP antibodies or with 10 µg/ml of BSA for measuring anti-BSA antibodies. Other test proteins were also coated at 10 µg/ml. Following treatment with alkaline phosphate-conjugated antibodies, microtiter plate wells were filled with 200 µl of p-nitrophenyl phosphate (1 mg/ml in 1 M Tris, pH 9.8), incubated for ½ to 1 hour at room temperature, and the A₄₀₅ of the solution in each well was determined with a Titertek Multiskan Spectrophotometer (Flow Laboratories, McLean, VA). The titers for the ELISA were calculated as previously described (See Current Protocols in Immunology, Vol. I, ed. J. Coligan, A. Kruisbeck, D. Margulies, E. Shevach and W. Strober. J. Wiley & Sons, 1991, specifically incorporated herein by reference).

### Example 1

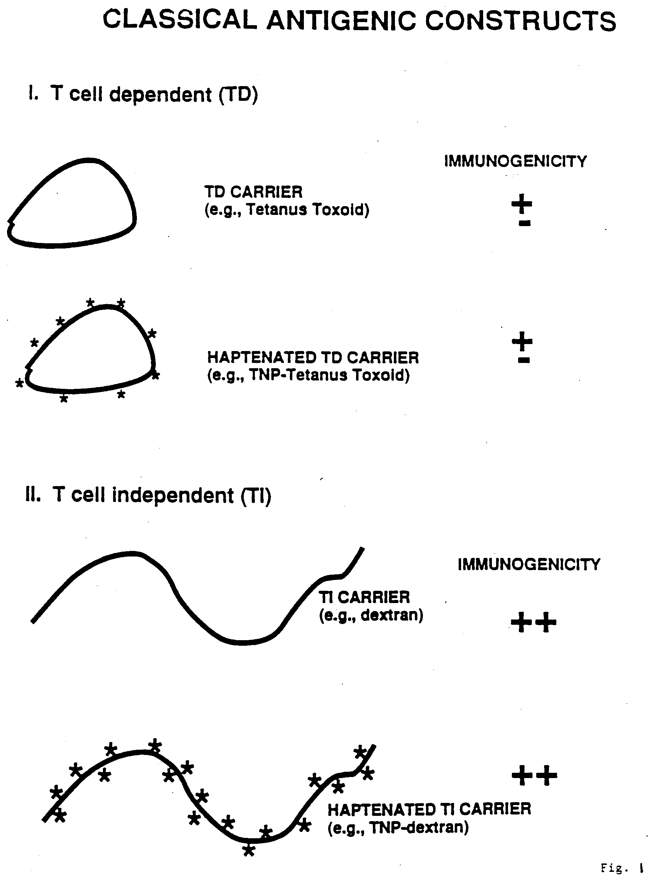

Current vaccines have many deficiencies that include poor immunogenicity, the need for separate vaccine constructs for each antigen and the requirement for multiple booster injections to elicit good antibody production. A high molecular weight polymer (HMWP) was utilized as a primary antigen carrier to provide the vaccine backbone. While any HMWP could be used, Dex was the choice for these studies. The Dex HMWP was used to provide a high density presentation of the secondary carrier to T cells and B cells and to provide a carrier for many other different antigens (Figures 1 & 2).

When antigens are coupled to the HMWP primary carrier the antibody response to these antigens is enhanced (Fig 3 and 4). While a single injection of unconjugated BSA elicits no detectable antibody response, BSA conjugated to Dex elicits a good antibody response at 10-500 µg/dose (Figure 3). Conjugation of different proteins to Dex, the primary carrier, makes them strongly immunogenic (Figure 4). These studies included viral antigens (Vaccinia-Dex), bacterial antigens and toxins (Cholera toxin-Dex) and other substances that were poorly immunogenic. Therefore, a secondary protein carrier could include a wide variety of proteins such as BSA or toxins/toxoids such as cholera, tetanus or diphtheria. The molecular weight of the Dex primary carrier can vary, but must be > 70 kDa and is a most effective carrier molecule at about 400 and 2000 kDa) in size (Figure 12). However, the optimal size for the HMWP may vary depending on the specific primary carrier utilized. The Dex HMWP is a polysaccharide and immune defective mice do not mount antibody responses to it. However, both normal and immune defective mice produce equally good antibody responses to BSA coupled to Dex (Fig. 10). This demonstrates that Dex as a primary carrier may simply provide a matrix to present antigen(s) to cells in a multivalent array and itself need not be immunogenic. These studies also show that a variety of proteins could be used as secondary carriers and that the secondary carrier could serve both as a vaccine antigen and a carrier for non-immunogenic antigens.

### Example 2

The dual carrier vaccine construct is demonstrated fully using TNP (Figure 5). Mice are immunized with TNP coupled to the secondary carrier BSA (TNP-BSA) and TNP-BSA is also coupled to the HMWP primary carrier (Dex). Conjugation of TNP to the secondary carrier BSA alone did not enhance the antibody response to TNP. However, coupling the secondary carrier/TNP complex to the primary carrier to provide [(TNP-BSA)-Dex] enhanced the immunogenicity of TNP. In addition BSA (the secondary carrier) was immunogenic when coupled with Dex alone or with Dex and TNP. Therefore using the dual carrier vaccine construct, a HMWP can carry the secondary carrier and other antigens may be coupled to the secondary carrier. Antibodies will be elicited to the secondary carrier as well as the antigen conjugated to it and non-immunogenic haptenic molecules will be rendered immunogenic. This could be particularly important if the secondary carrier is an antigen to which antibody would provide protective immunity, such as tetanus or diphtheria toxoid. This vaccine construct also allows multiple unrelated antigens to be conjugated to the primary carrier (Figure 7). TNP was coupled to OVA (the secondary carrier) and then conjugated to Dex ( the primary carrier). LYS was also independently conjugated directly to Dex. Antibodies were elicited to each of the antigens. Thus the HMWP backbone of this vaccine construct is suitable for producing multivalent vaccines with an array of secondary carrier proteins and or many different antigens coupled to the secondary carrier. (multicarrier/multiantigen vaccine)

### Example 3

In the previous examples the dual carrier vaccine construct was shown to be effective with a variety of antigens. This data is supported and extended using a parasitic (malarial) derived peptide antigen (p74). The dual carrier vaccine construct was shown to be significantly better than peptide antigen alone or peptide conjugated to the secondary carrier BSA (Figure 6). A good antibody response was elicited to this small antigen only after the P74-BSA conjugate was coupled to the HMWP primary carrier (Dex). However, the HMWP carrier simply provides a matrix to present antigens to cells and need not be immunogenic (Fig. 10).

### Example 4

For a vaccine to effectively enhance immunity over a long period of time, a good booster response is important. After a primary immunization with the dual carrier vaccine, a booster antibody response can be elicited even with the unconjugated secondary carrier (Figure 8). Furthermore, when a haptenated carrier was employed a booster response was observed to both the TNP and to the BSA secondary carrier. Further analysis shows that the primary and secondary antibody response to the BSA secondary carrier is long lived (Figure 9).

### Example 5

The BSA-Dex conjugate was analyzed to evaluate the effect of host immune status and route of immunization on antibody response. Both immunologically mature adult and immunologically immature baby mice were effectively immunized with the primary and secondary carrier complex demonstrating that the dual carrier vaccine will elicit an antibody response even in babies and young infants with immature immunity (Figure 11). The antibody response to BSA (the secondary carrier) is similar in both the adult and baby mice. In addition IV, IM, and SC routes all elicited a good antibody response to BSA (Figure 13). The route of administration of vaccine is not limited therefore to any single type of inoculation and it is not limited by the age or immunologic status of the vaccine recipient. However, the size of the HMWP is critical to provide an effective vaccine construct. For example for the HMWP Dex the size must be >70,000D, preferably >400,000D (Fig. 12).

### Example 6

The preparation of a multiple carrier vaccine is illustrated in Fig. 14. Three secondary carriers are employed in this system, two of which are further conjugated to another moiety. H. influenzae PRP is coupled to a tetanus toxoid secondary carrier, a malarial derived peptide is coupled to a meningococcal outer membrane protein, and a viral protein (such as RSV-F protein) is left unconjugated. One or more of each of the secondary carriers is then conjugated to the high molecular weight polymer backbone.

### Example 7

A vaccine can be designed to bear multiple specificities under any of the following four approaches:
(1) Two or more constructs, each consisting of different secondary carriers conjugated to the same primary carriers;
(2) Two or more different constructs, each consisting of the same primary and secondary carrier but different moieties;
(3) Two or more different constructs, each consisting of different primary carriers, the same secondary carriers, and different moieties; and
(4) Two or more different constructs, each consisting of different primary and secondary carriers and different moieties.

### Example 8

As set forth in Charts 2 and 3 below, immunization with dextran-conjugated to TNP-BSA greatly enhanced many parameters of the antibody response, including magnitude, persistence of response etc. (Chart 2). Similarly, immunization with BSA-dextran or TNP-BSA dextran greatly enhanced many parameters of the antibody response (Chart 3).

| Chart 2 **Comparison of antibody responses elicited by TNP-dextran and BSA-dextran or TNP-BSA dextran** | | |
|---|---|---|
| | immunized with: | |
| | TNP-BSA (no adjuvant) | TNP-BSA dextran |
| | anti-TNP titers | |
| A. Magnitude response | ― | ++++ |
| B. Persistence response | ― | ++++ |
| C. Ability to boost for secondary response | ― | ++++ |
| D Ability to immunize neonate | ― | ++++ |
| E. Ability to immunize B cell defective mice | ― | ++++ |
| F. Antibody activity | ― | ++++ |
| G. Ability to immunize subcutaneously | ― | ++++ |

| Chart 3 **Comparison of antibody responses elicited by TNP-dextran, BSA-dextran or TNP-BSA dextran** | | |
|---|---|---|
| | immunized with: | |
| | TNP-dextran anti-TNP titer | BSA-dextran or TNP-BSA dextran anti TNP or BSA titer |
| A. Magnitude response | + | ++++ |
| B. Persistence response | 7-10 d | 40-60 d |
| C. Ability to boost for secondary response | ― | ++++ |
| D Ability to immunize neonate | ― | ++++ |
| E Ability to immunize B cell defective mice | ― | ++++ |
| F. Antibody activity | Low | High |
| G. Ability to immunize subcutaneously | + | ++++ |

### Example 9

Three dual carrier constructs were prepared with dextran as the primary carrier and diptheria toxoid (DT), pertussis toxoid (PT), and tetanus toxoid (TT) as secondary carriers. These conjugates were first analyzed for dextran and protein concentrations to assess success of conjugation. Thereafter, the conjugates were injected into two groups of mice to determine the immune response to the dual carrier immunogenic constructs.

### A. Materials and Methods

### 1. Animals

Female outbred mice (CD-1), 4 weeks old, were purchased from Charles River, Wilmington, MA.

### 2. Reagents

Hyperimmunized anti-TT mouse serum was received from Dr. S.C. Szu, Laboratory of Developmental and Molecular Immunity, NIH, Bethesda. Purified mouse IgG1, IgG2a, IgG2b, IgG3 and IgM as well as alkaline phosphatase conjugates of goat anti-mouse IgG, IgG1, IgG2a, IgG2b, IgG3, IgM were from Fisher Biotechnology, Springfield, NJ. Purified mouse IgG, purified goat anti-mouse IgG (Fab specific), bovine serum albumin (BSA), avidin from egg white, Brij-35 and phosphatase substrate were from Sigma Chemical Co., St. Louis, MO. Chromatographically purified tetanus toxoid (TT), pertussis toxoid (PT) and diptheria toxoid (DT) were provided by the Massachusetts Public Health Biological Laboratories (MPHBL) both for preparing the secondary carriers as well as for coating ELISA plates as described below.

Anti-dextran reference mouse serum was prepared by injecting mice with dextran-pertussis toxoid (PT) conjugate (prepared as described below) twice at an interval of 31 days and bleeding mice 2 weeks after the second injection. The sera of various mice were pooled in equal volumes. Similarly, anti-DT reference mouse serum and anti-PT reference mouse serum were prepared by injecting mice with aluminum phosphate adsorbed (adsorption process described below) DT or PT.

### B. Preparation of Secondary Carrier Proteins

MPHBL prepared the secondary carrier proteins, DT, PT, and TT, by the following purification and detoxification techniques:

Tetanus toxoid was prepared by first purifying tetanus toxin by ammonium sulphate fractionation and gel filtration through Sephadex G-50 and DEAE column chromatography. Purified tetanus toxin was then detoxified with formalin. Diptheria toxoid was prepared by first detoxifying diphtheria toxin with formalin and then purifying DT by ammonium sulphate fractionation and DEAE column chromatography. Pertussis toxoid was prepared by purifying pertussis toxin by affinity chromatography and then detoxifying pertussis toxin with tetra nitro methane.

Using a Centricon 30 device (from Amicon), diptheria toxoid was concentrated to 10 mg/ml, and tetanus toxoid to 4.7 mg/ml, and the pertussis is toxoid to 2.8 mg/ml.

A 1/9 volume of HEPES buffer concentrate (0.75 M HEPES, 10 mM EDTA, pH 7.5) Was added to each toxoid solution and a 12-14 molar ratio of SATA (Calbiochem) slowly added from a 25 mM or 0.1 M stock in dimethyl formamide (DMF). Perhaps because many of the readily accessible amino groups were blocked during the detoxification process, higher ratios of reagent to protein than is described above under Methods was necessary in order to obtain good yields of conjugate. After one hour at room temperature, the derivatized toxoids were exchanged into acetate buffer on a P6DG column (BioRad) and the protein in the void volume concentrated using a Centricon 30. Before the pertussis toxoid could be concentrated, it was initially cleared of cloudiness by the addition of 1/9 volume of HEPES buffer to raise the pH. In a subsequent preparation, the pertussis toxoid was exchanged into HEPES buffer at pH 7.5. The solution remained clear.

### B. Preparation of the Primary Carrier

The primary carrier was prepared as described above in Methods. In brief, high molecular weight amino ethyl carbamyl dextran (c.a. 2 million daltons), lightly radiolabeled with tritium, was iodoacetylated with SIAP and exchanged into acetate buffer (10 mM sodium acetate, 2 mM EDTA, 0.1 M NaCl, 0.02% sodium azide) and concentrated.

### C. Conjugation of Toxoids to Dextran

The conjugates were prepared using a variation of heteroligation chemistry referred to above (Brunswick et al., Mongini et al.) In brief, the acetyl-thiolated toxoid was added to a gently vortexed solution of the iodoacetylated dextran. The pH was raised and the thiol deprotected by the addition of approximately 1/9 volume of concentrated HEPES buffer containing 0.5 M hydroxylamine. The solution contained approximately equal amounts of toxoid and dextran on a weight basis. The final toxoid concentrations were 5 mg/ml (DT), 1.1 mg/ml (PT) and 5.9 mg/ml (TT).

After an overnight reaction at 4°C, the conjugates were treated for 1 hr with 0.2 mM mercaptoethanol followed by 10 mM iodoacetamide for 10 min. The conjugates were then fractionated by gel filtration on either a S300SF or a S400SF 1 x 58 cm column (Pharmacia), equilibrated with PBS. The void volume peak containing the conjugate was then pooled and sterile filtered using a Millex HV (0.45 µm) filter (Millipore).

Of the three toxoids, pertussis responded particularly well to the conjugation protocol. As set forth in Fig. 15, a high percentage of pertussis toxoid converted to high molecular weight conjugate with dextran. It is estimated that a maxiumum of 15 amino groups, and probably many fewer, were modified on the toxoid.

### D. Determination of the Success of the Conjugation

Protein concentrations on each of the three conjugates were determined by OD 280 and confirmed by the micro BCA assay (Pierce) using the purified toxoids obtained from MPHBL as standards. Dextran concentrations were determined from the specific activity of the tritiated AECM dextran. These results are set forth below:

| | Protein (mg/ml) | Protein/dex (Molar) | Protein/dex (w/w) |
|---|---|---|---|
| 1. Dtxd-dex | 0.34 | 12 | 0.6 |
| 2. Ptxd-dex | 0.73 | 14 | 0.7 |
| 3. Ttxd-dex | 0.43 | 22 | 1.1 |

The weight/weight ratio of protein to dextran indicates that the conjugation methods successfully coupled the toxoids to the dextran. These results were confirmed by analysis on Ouchterlony plates.

### D. Adsorption of conjugates onto aluminum phosphate

In addition to fluid conjugates corresponding to each toxoid, samples of each conjugate were adsorbed onto the adjuvant aluminum phosphate. Each sample was adsorbed onto 4 mg/ml of freshly prepared aluminum phosphate gel (pH 6.0) made by precipitation of aluminum chloride and trisodium phosphate. Thimerosal was added at a final concentration of 0.01% (w/v). The preparations were shaken overnight at room temperature.

### E. Immunization of Mice

Two sets of immunogenicity experiments were carried out.
1. For the first experiment, groups of 5 mice were injected with various conjugates as fluid or adsorbed onto the adjuvant aluminum phosphate. The does of various conjugates for mice were selected on the basis of protein dose which would produce antibody response to proteins in mice. Figure 16 shows the doses of various conjugates injected to mice. The same doses were used for fluid and aluminum phosphate adsorbed conjugates. For the adsorbed preparations, the dose of aluminum phosphate for each mouse was 0.4 mg/ml, which is 1/5th of the human dose of adjuvant used at the MPHBL for adsorbed tetanus toxoid. Each mouse was injected subcutaneously with 0.1 ml of fluid or adsorbed conjugate diluted to appropriate concentrations.
   The mice were bled at 2, 4 and 8 weeks and sera separated. The sera for a group were pooled in equal volumes.
2. The second experiment was performed to study the immune response of mice to the same dose of dextran present in various conjugates and to assess the effect of a booster dose of the conjugate. Groups of 10 mice were injected twice, separated by an interval of 31 days, subcutaneously with 2 µg per mouse of dextran in various conjugates or free dextran as set forth on Figure 19a. The mice were bled 4 weeks after the first injection and 2 weeks after the second injection. The sera were separated and the individual mouse sera were assayed for anti-dextran or anti-protein antibodies by ELISA, as described below.

### F. Enzyme linked immunosorbent assay

The anti-dextran reference mouse serum was standardized for respective antigen specific IgG, IgM and IgG isotypes. The hyperimmunized anti-tetanus toxoid reference mouse serum, anti-diphtheria toxoid reference mouse serum and anti-pertussis toxoid reference mouse serum were standardized for respective antigen specific IgG by the method set forth in W.D. Bollinger and J.W. Boslego, A general approach to standardization of the solid-phase immunoassay for quantitation of class-specific antibodies, J. Immunol. Methods 1981, 46, 129-140 with modification.

ELISA was performed on high binding easy wash microtiter plates (Corning Glass Works, Corning, NY) coated with 100 µl of particular antigen, here TT, PT or DT, diluted to 5 µg/ml in phosphate buffered saline (PBS), pH 7.2 at RT for overnight. The plates were washed between all steps with PBS containing 0.05% between 20. Serial two-fold dilutions of reference serum and test sera in PBS containing 0.1% Brij 35 and 0.5% BSA (PBB) were done in plates. The plates were held at room temperature for 2 hours and washed. The alkaline phosphatase labelled goat anti-mouse IgG, IgG1, IgG2a, IgG2b, IgG3 or IgM diluted in PBS were added to respective plates. The plates were again held at room temperature for 2 hours and washed. Finally 100 µl phosphatase substrate diluted to 1 mg/ml in 1 M diethanolamine-0.5 mM magnesium chloride buffer, pH 9.8, was added to each well. The plates were read at 405 nm wavelength on an ELISA reader (Titertek Multiskan Plus, Flow Laboratories). Antigen specific antibodies (µg/ml) were extrapolated from the standard curve using Rodbard's transformation. For anti-dextran ELISA, initially plates were coated with avidin overnight and then 1 µg/ml solution of biotinylated dextran in PBS was added at room temperature for 1 hour. This ELISA gave very similar results of anti-dextran IgG for 30 serum pools to that when plates were coated with dextran directly. Therefore, most experiments were carried out by coating the plates with dextran directly.

### G. Results

### 1. The Single Injection

Unlike the mice used in the prior examples, this experiment focused on young mice that, as set forth in the Introduction and Figure 11, do not respond well to unconjugated dextran and other T-independent antigens. As those in the art know, young children under two years of age also do not respond well to type 2 T-independent antigens. These results, therefore, are more directly applicable to young children.

These mice elicited antibodies by two weeks and persisted at high levels for at least 8 weeks. As set forth in Fig. 16, the main isotypes elicited were IgG1, IgG3, and IgGM. In addition, mice injected with PT-dex elicited low levels of IgG2a and IgG2b. Fig. 16 also demonstrates that the mice that received aluminum phosphate absorbed conjugates exhibited higher levels of IgG and IgM antibodies than those that received the fluid preparations.

In addition, the antibody responses to the secondary carriers, DT, TT, and PT, were evaluated and set forth in Fig. 17 (a), (b), and (c). In each case, the mice that received the conjugates exhibited antibodies to the protein component by at least two weeks and persistence of those antibodies for at least eight weeks. Similarly, absorbtion to aluminum phosphate enhanced the immune response.

Moreover, these data clearly demonstrate an anamnestic response for each component of the dual conjugate.

As expected with the immunogenic tetanus toxoid, there was an antibody response to both conjugated and unconjugated forms. With the poorly immunogenic diptheria and pertussis toxoids, however, there was marked enhancement of the immune response to the conjugated form.

Together, Figs. 16 and 17 demonstrate the dual and reciprocal immune enhancement achieved with the conjugates of the invention.

More significantly, the neutralizing activity of the tetanus toxoid was evaluated as set forth in Fig. 18. A blood level of 0.01 anti-toxin units (Au) or greater is considered to provide protection against tetanus toxin. Thus, the conjugate elicited protective levels of antibody against tetanus toxin within four weeks for the fluid and two weeks for the conjugate absorbed on the aluminum phosphate adjuvant at doses of 0.1 and 1.0 µg.

### 2. The Booster Injection

As set forth in Fig. 19(a), constant amounts of dextran to varying amounts of protein were injected into the mice. This chart also demonstrates that each conjugate elicited an immune response against the secondary carrier (the toxoid). Fig. 19(b) shows that unconjugated dextran alone elicits very low levels of IgG3 and IgM after two doses as compared to the anti-dextran antibody levels elicited by various conjugates. As observed in the first experiment, dextran-PT conjugate induced relatively greater levels of IgG2a and IgG2b than other conjugates.

Together, parts (a) and (b) of Fig. 19 demonstrate the dual and reciprocal immune enhancement achieved with the conjugates of the invention, i.e., conjugation of the secondary carrier provided enhanced response to the primary carrier and vice-versa.

### Example 10

The following illustrates the synthesis of the dual carrier immunogenic construct with diptheria, pertussis, tetanus toxoid, which are clinically relevant protein secondary carriers, conjugated to the high molecular weight primary carrier haemophilus influenzae type b carbohydrate (polyribosyl riboitol phosphate "PRP"), which is also of clinical relevance. Note that all individual components have already been approved for human use.

### A. Preparation of the Secondary Carriers

Toxoids were concentrated using a Centricon 30. Diptheria toxoid (DT) was concentrated to 5.25 mg/ml, pertussis toxoid (PT) to 2.6 mg/ml and tetanus toxoid (TT) to 3.2 mg/ml. A 1/9 volume of a concentrated HEPES buffer added. SPDP (available from Sigma or Calbiochem) was added to a gently vortexed solution of DT at a molar excess of 16:1, and to TT at a molar excess of 20:1. For PT, the reagent SATA at an 11:1 ratio (from a stock in DMF) was added to a gently vortexed solution.

The two reagents, SATA and SPDP, yield different protected thiols, with different advantages known to those in the art. In brief, the product of SATA is an acetyl thiol, which is deprotected slowly with hydroxylamine, while the product of SPDP is a thio-pyridyl group. The latter is rapidly removed with DTT to yield a product with an absorbance, permitting easy quantitation of the number of thiol groups. SATA may be the reagent of choice when the carbohydrate is iodoacetylated, since the thiol deprotecting reagent, hydroxylamine, is compatible. SPDP may be preferred when the carbohydrate is derivatized with maleimide and the deprotecting reagent must be removed prior to conjugation.

After 1-2 hours, the solutions of DT and TT were exchanged into acetate buffer on a P6DG column, concentrated and made 50 mM in dithiothretol (DTT) for 20 min. The PT solution was made 50 mM in hydroxylamine for 30 min and then desalted on a P6DG column equilibrated with HEPES buffer, as PT may precipitate at low pH. Thiolated toxoids were concentrated with a Centricon 30. The molar ratio of thiol to toxoid was 6.5 for DT and 4.7 for TT. It was not determined for PT.

### B. Preparation of the Primary Carrier

High molecular weight haemophilus influenza type b carbohydrate (polyribosyl riboitol phosphate = PRP) was derivatized with adipic dihyrazide (PRP-AH) using the cyanogen bromide method followed by size fractionation by gel filtration. (Cruse J.M., Lewis R.E. Jr. ed., Conjugate vaccines in Contributions to Microbiology and Immunology, vol. 10, 1989). The final product had an average molecular weight of 300 kDa and approximately 16 hydrazide groups/300 kDa of carbohydrate. The PRP-AH was a gift of the Massachusetts Public Health Biological Laboratories (MPHBL), Boston, Mass.

The PRP-AH was further derivatized according to the following method. Higher concentrations of crosslinking reagent and/or longer incubation times were used to compensate for the reduced reactivity of the hydrazide group compared with that of the amino moiety of AECM-dextran.

For most preparations, the PRP-AH was made thiol reactive by maleylation with the reagent GMBS (Calbiochem) instead of by iodoacetylation (with SIAP) because the maleimide concentration can easily be determined from its absorbance at 304 nm (extinction coefficient - 620 M⁻¹ (Kitagawa, T., et al. J. Biochem. 94:1160 (1983)). Additionally, the maleimide group is (1) more reactive than the iodoacetyl group, permitting shorter reaction times and (2) hydrolyzes and so does not necessarily need to be quenched with mercaptoethanol, an advantage for thiol sensitive proteins. The disadvantage of the maleimide group is its relative bulk, which could introduce additional epitopes, and that hydrolysis reduces the number of active groups, which could decrease the extent of cross-linking. However, particular reagents and conditions were selected to improve stability (e.g., an alkyl maleimide, high reagent concentration and short reaction time and pH 5 desalting step). Conjugates with PRP-AH have also been prepared using the iodoacetylating reagent, SIAP, as was used with the dextran conjugates.

An example of maleimide-PRP is as follows. 40 µl of the maleimide reagent GMBS (0.1 M in DMF) (CalBiochem) was added to a vortexed solution of 2 mg PRP-AH in 157 µl of HEPES buffer (final concentration: 75 mM HEPES, 1 mM EDTA, 0.02% azide, pH 7.5). After 2 h at room temperature, the solution was desalted on a P6DG column (BioRad) equilibrated with acetate buffer (10 mM sodium acetate, 0.1 M NaCl, 2 mM EDTA, 0.02% sodium azide) and the void volume concentrated to 0.47 ml with a Centricon 30 (Amicon). Using an extinction coefficient of 620 M⁻¹ at 304 nm, the maleimide concentration was estimated to be 225 µM. The residual hydrazide content was estimated using a TNBS assay (extinction coefficient = 17400 M⁻¹ at 498 nm for hydrazides), to be less than 4 µM. Thus, derivatization was judged to be essentially complete. In the preparation of the conjugate with pertussis toxoid, the derivatized PRP was desalted with a column equilibrated with HEPES buffer at pH 7.5 instead of acetate.

### C. Conjugation of the Secondary Carriers to PRP

Thiolated DT and TT were added to stirred solutions of deoxygenated PRP-maleimide under a nitrogen atmosphere and the pH raised by the addition of 1/9 volume of concentrated HEPES buffer and left to stir overnight at room temperature. Thiolated PT was added to a vortexed solution of PRP-maleimide and left overnight at 4°C. After the overnight reaction, solutions were made 0.2 mM mercaptoethanol for one hour (to ensure complete removal of unhydrolyzed maleimide) followed by 10 min in 10 mM iodoacetamide (to consume all free thiols). Solutions were fractionated by gel filtration on a S300SF or a S400SF column to remove unconjugated PRP and toxoid. The high molecular weight conjugate was concentrated with a Centricon 30 and sterile filtered.

As set forth in Fig. 20, diphtheria toxoid very successfully converted to a high molecular weight conjugate with PRP. It is estimated that less than seven amino groups were modified on the toxoid.

The composition of the final products were:

| Toxoid | Toxoid/PRP (molar) (for PRP = 300kDa) | Toxoid (wgt/wgt) |
|---|---|---|
| DT | 4.5 | 0.8 |
| PT | 5.4 | 2.9 |
| TT | 3.9 | 1.9 |

These data, particularly the weight/weight measurements, demonstrate the success of the conjugation.

We believe that this method differs from other known conjugates in (1) details of the chemistry, principally the use of a thio-ether linkage and (2) the use of a high molecular weight PRP, (3) the use of chromatographically purified toxoids and (4) in particular, the use of an accellular pertussis toxoid.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

## Claims

1. A dual carrier immunogenic composition, comprising at least one primary carrier and at least one secondary carrier, wherein said primary carrier is a molecule having a molecular weight equal to or greater than 400 kDa and having T-independent antigen characteristics, said secondary carrier is a T-dependent antigen and is chemically conjugated to said primary carrier, wherein the composition enhances an antibody response to the primary carrier and to the secondary carrier.

2. The dual carrier immunogenic composition of claim 1, wherein at least one primary carrier is selected from the group consisting of dextran and a microbial polysaccharide.

3. The dual carrier immunogenic composition of claim 2, wherein at least one primary carrier is dextran.

4. The dual carrier immunogenic composition of claim 2, wherein at least one primary carrier is a microbial polysaccharide.

5. The dual carrier immunogenic composition of claim 4, wherein at least one primary carrier is *H. influenzae* type b polyribosyl-ribitol-phosphate.

6. The dual carrier immunogenic composition of claim 4, wherein at least one primary carrier is a pneumococcal capsular polysaccharide.

7. The dual carrier immunogenic composition of claim 1, wherein at least one secondary carrier is a protein or a peptide.

8. The dual carrier immunogenic composition of claim 7, wherein the protein or peptide is selected from the group consisting of viral, bacterial, parasitic, animal and fungal proteins or peptides.

9. The dual carrier immunogenic composition of claim 8, wherein the protein or peptide is selected from the group consisting of diphtheria toxoid, pertussis toxoid, tetanus toxoid, bacterial outer membrane protein, viral outer membrane protein, and combinations thereof.

10. The dual carrier immunogenic composition of claim 1, wherein at least one primary carrier is chemically conjugated to one or more secondary carriers.

11. The dual carrier immunogenic composition of claim 1, further comprising an adjuvant.

12. A vaccine, comprising:
an immunostimulatory amount of at least one of the dual carrier immunogenic compositions of claim 1, and
a pharmaceutically acceptable carrier.

13. Use of the vaccine of claim 12 for the preparation of a medicament for the treatment of a patient wherein the vaccine is for administering to the patient in an immunostimulatory amount.

14. The use of claim 13, wherein the vaccine is for administering intravenously, intramuscularly, or subcutaneously.

15. A method of preparing antibodies against diseases caused by bacteria, rickettsiae, viruses, parasites, fungi or chemicals, comprising the steps of:
immunizing a host with the vaccine of claim 12 to produce antibodies directed against the immunogen, and
isolating the antibodies or B cells from the donor.

16. An immunotherapeutic composition, comprising the antibodies prepared by the method of claim 15.

17. The immunotherapeutic composition of claim 16, wherein the B cells are used to produce monoclonal antibodies.

18. Use of the immunotherapeutic composition of claim 16 for the preparation of a medicament for the treatment of a patient wherein the composition is for administering to the patient in a therapeutically effective amount.
